# EUROPEAN PATENT APPLICATION

(11) **EP 2 789 630 A1**
(43) Date of publication of application: **15.10.2014**
(21) Application number: 13001840.1
(22) Date of filing: 09.04.2013
(51) Int. Cl.: C07K 16/28, C07K 16/30, C07K 16/40, A61K 39/395, A61P 35/00

(54) **Bispecific antibodies against CD3e and ROR1**

(71) Applicant: EngMab AG, 8832 Wilen (CH)
(72) Inventor: Vu, Minh Diem, 8832 Wollerau (CH); Strein, Klaus, 69469 Weinheim (DE)
(74) Representative: Schreiner, Siegfried

(57) **Abstract**

A bispecific antibody specifically binding to the two targets human CD3ε (further named also as "CD3") and the extracellular domain of human ROR1 (further named also as "ROR1"), characterized in that the bispecific antibody does not internalize in a cell based assay at 37°C during 2 hrs., using ROR1-positive B-CLL cells and used at an antibody concentration of 1 nM, the Δ mean fluorescence intensity (ΔMFI), MFI as measured by flow cytometry, reflecting the internalization (%) of the said antibody to ROR1-positive cells is between 120% to 0%, preferably from 100% to 0%, of the ΔMFI (internalization, %) of a defined anti-ROR1 antibody and which is useful for the treatment of B-cell malignancies like Chronic Lymphocytic Leukemia or plasma cell disorders like Multiple Myeloma MM or other B-cell disorders expressing ROR1 and ROR1-positive solid tumors.

## Description

The present invention relates to novel bispecific antibodies against CD3ε and ROR1, their manufacture and use.

### Background of the Invention

ROR1 (synonyms: tyrosine-protein kinase transmembrane receptor ROR1, EC=2.7.10.1, neurotrophic tyrosine kinase, receptor-related 1, UniPrtKB Q01973) is a tyrosine-protein kinase receptor. The receptor is described in Masiakowski P., Carroll R.D., J. Biol. Chem. 267:26181-26190(1992) "A novel family of cell surface receptors with tyrosine kinase-like domain." WO9218149 and WO9527060 mention ROR-1 as Rtk-2 and antibodies against ROR-1. WO2002087618 mentions a method of controlling the growth and differentiation of cancer by selectively inhibiting a growth factor receptor. Such a receptor would be Ror1 or Ror2. WO2005100605 mentions ROR1 as a therapeutic target for breast cancer and anti ROR1 antibodies which specifically bind to ROR1, to the extracellular region of ROR1 (M1-V406) and ROR1 fragments Q73-V139, E165-I299, K312-C391. WO2007051077 relates to an anti-ROR1 antibody and its use in lymphoma cell detection. WO2008103849 also mentions anti-ROR1 antibodies. Rabbani (Blood (ASH Annual Meeting Abstracts) 2010 116: Abstract 916) discloses the use of anti ROR1 antibodies for the treatment of chronic Lymphocytic leukemia (CLL). Rabbani used anti-ROR1 an antibody against the extracellular domain, an antibody against the CRD region (ligand binding site for Wnt proteins) and an antibody against the kringle domain. Daneshmanesh AH et al., Int. J. Cancer, 123 (2008) 1190-1195 relates to an anti ROR1 antibody that binds to the extracellular domain fragment WNISSELNKDSYLTL (SEQ ID NO:18) and an anti ROR1 antibody that binds to the intracellular domain fragment KSQKPYKIDSKQAS (SEQ ID NO:20). Also the use of such antibodies for the treatment of CLL is mentioned.

WO2011159847 relates to an anti-ROR1 antibody as a conjugate with a biologically active molecule for the treatment of ROR1 cancer like lymphoma or adenocarcinoma. WO2008076868, WO2008103849, WO201008069, WO2010124188, WO2011079902, WO2011054007, WO2011159847, WO2012076066, WO2012076727, WO 2012045085, and WO2012097313 relate also to ROR1 binding molecules or anti ROR1 antibodies. WO2012075158 relates to an anti-ROR1 antibody comprising as light chain variable domain (VL) the sequence of SEQ ID NO:2 and as variable heavy chain domain (VH) the sequence of SEQ ID NO:6, and as respective CDRs the sequences of SEQ ID NO: 3, 4, 5, 7, 8, 9.

WO2005040413 is directed to a screening method for the identification and/or validation of inhibitors of a receptor tyrosine kinase activity, including ROR1.

WO2008036449, WO2011014659 and WO2011050262 mention bispecific antibodies wherein one target can be ROR1. WO2007146968 mention multivalent single-chain binding proteins with effector function and ROR1 and CD3 are mentioned as possible targets. WO2011054007 is directed to a method of treatment of cancer administering an affinity reagent which binds to the extracellular domain of ROR1. Bispecific antibodies with CD3 are also mentioned.

The TCR/CD3 complex of T-lymphocytes consists of either a TCR alpha (α)/beta (β) or TCR gamma (γ)/delta (δ) heterodimer coexpressed at the cell surface with the invariant subunits of CD3 labeled gamma (γ), delta (δ), epsilon (ε), zeta (ζ), and eta (η). Human CD3ε is described under UniProt P07766 (CD3E_HUMAN). An anti CD3ε antibody described in the state of the art is SP34 (Yang SJ, The Journal of Immunology (1986) 137; 1097-1100). SP34 reacts with both primate and human CD3. SP34 is available from Pharmingen. A further anti CD3 antibody described in the state of the art is UCHT-1 (see WO2000041474). A further anti CD3 antibody described in the state of the art is BC-3 (Fred Hutchinson Cancer Research Institute; used in Phase I/II trials of GvHD, Anasetti et al., Transplantation 54: 844 (1992)). SP34 differs from UCHT-1 and BC-3 in that SP-34 recognizes an epitope present on solely the ε chain of CD3 (see Salmeron et al., (1991) J. Immunol. 147: 3047) whereas UCHT-1 and BC-3 recognize an epitope contributed by both the ε and γ chains. The sequence of an antibody with the same sequence as of antibody SP34 is mentioned in WO2008119565, WO2008119566, WO2008119567, WO2010037836, WO2010037837 and WO2010037838. A sequence which is 96% identical to VH of antibody SP34 is mentioned in US8236308 (WO2007042261). VH and VL sequences of a further antibody with the same sequences as of SP34 are shown in SEQ ID NO:10 and 11.

A wide variety of recombinant bispecific antibody formats have been developed in the recent past, e.g. by fusion of, e.g. an IgG antibody format and single chain domains (see Kontermann RE, mAbs 4:2, (2012) 1-16). Bispecific antibodies wherein the variable domains VL and VH or the constant domains CL and CH1 are replaced by each other are described in WO2009080251 and WO2009080252.

WO2013026831 provides a bispecific antigen binding molecule, comprising a first Fab fragment which specifically binds to a first antigen, a second Fab fragment which specifically binds to a second antigen, and an Fc domain. WO2013026833 provides a T cell activating bispecific antigen binding molecule comprising a first and a second antigen binding moiety, one of which is a Fab molecule capable of specific binding to an activating T cell antigen and the other one of which is a Fab molecule capable of specific binding to a target cell antigen, and an Fc domain.

WO2013026835 relates to bispecific antibodies comprising at least two Fab fragments, wherein the first Fab fragment comprises at least one antigen binding site specific for a first antigen; and the second Fab fragment comprises at least one antigen binding site specific for a second antigen wherein either the variable regions or the constant regions of the second Fab heavy and light chain are exchanged; and wherein the bispecific antibody is devoid of a Fc domain. Said antibody can additionally comprise a third Fab fragment. Said third Fab fragment can comprise at least one antigen binding site specific for the first or second antigen, preferably for the first antigen. WO2013026837 provides a T cell activating bispecific antigen binding molecule comprising a first and a second single chain Fv (scFv) molecule fused to each other, wherein the first scFv molecule is capable of specific binding to a target cell antigen and the second scFv molecule is capable of specific binding to an activating T cell antigen; **characterized in that** the T cell activating bispecific antigen binding molecule further comprises an Fc domain composed of a first and a second subunit capable of stable association.

WO2013026839 relates to bispecific antibodies that specifically bind a T-cell activating antigen and a Tumor Antigen (TA), comprising a first Fab fragment and a second Fab fragment, wherein either the variable regions or the constant regions of the second Fab heavy and light chain are exchanged; and wherein the bispecific antibody does not comprise a Fc domain. In one aspect of WO2013026839, a bispecific antibody that specifically binds a T-cell activating antigen and a Tumor Antigen (TA) is provided, comprising at least two fab fragments, wherein the first Fab fragment comprises at least one antigen binding site specific for a Tumor Antigen (TA); and the second Fab fragment comprises at least one antigen binding site specific for a T-cell activating antigen, wherein either the variable regions or the constant regions of the second Fab heavy and light chain are exchanged; and wherein the bispecific antibody is devoid of a Fc domain. WO2013026839 relates also to bispecific antibodies wherein the T-cell activating antigen is a CD3 T-Cell Co-Receptor (CD3) targeting antigen. In one embodiment the first and second Fab fragments are connected via a peptide linker. Preferably said peptide linker is a (G4S)₂ linker.

### Summary of the Invention

The invention relates to a bispecific antibody specifically binding to the two targets human CD3ε (further named also as "CD3") and the extracellular domain of human ROR1 (further named also as "ROR1"), **characterized in that** the bispecific antibody does not internalize in a cell based assay at 37°C during 2 hrs., using ROR1-positive primary B-CLL cells, and used at an antibody concentration of 1 nM, the Δ mean fluorescence intensity MFI (ΔMFI), as measured by flow cytometry, reflecting the internalization (%) of the said antibody to ROR1-positive cells is between 120% to 0%, preferably from 100% to 0%, of the ΔMFI (internalization, %) of an anti-ROR1 bivalent antibody of human IgG1 kappa (κ) type comprising as light chain variable domain (VL) the sequence of SEQ ID NO:2 and as variable heavy chain domain (VH) the sequence of SEQ ID NO:6, in the same concentration and experimental conditions.

In a further embodiment the invention relates to a bispecific antibody specifically binding to the two targets human CD3ε (further named also as "CD3") and the extracellular domain of human ROR1 (further named also as "ROR1"), **characterized in that** the bispecific antibody does not internalize in a cell based assay at 37°C during 2 hrs., using EHEB B-CLL cell line DSMZ ACC-67, and used at an antibody concentration of 1 nM, the Δ mean fluorescence intensity (ΔMFI), MFI as measured by flow cytometry, reflecting the internalization (%) of the said antibody to ROR1-positive cells is between 120% to 0%, preferably from 100% to 0%, of the ΔMFI (internalization, %) of an anti-ROR1 bivalent antibody of human IgG1 kappa (κ) type comprising as light chain variable domain (VL) the sequence of SEQ ID NO:2 and as variable heavy chain domain (VH) the sequence of SEQ ID NO:6, in the same concentration and experimental conditions.

Preferably the bispecific antibody according to the invention is devoid of a Fc domain.

Preferably the bispecific antibody according to the invention is characterized in comprising an anti-ROR1 antibody specifically binding to ROR1, and a Fab fragment or a single chain Fv fragment of an antibody specifically binding to CD3.

Preferably the bispecific antibody according to the invention is a bivalent antibody and characterized in comprising a monovalent anti-ROR1 antibody specifically binding to ROR1, and a monovalent antibody specifically binding to CD3. A bivalent antibody is preferred if its said ΔMFI (internalization, %) is between 100% and 0% of the ΔMFI (internalization, %) of the said anti-ROR1 bivalent antibody of human IgG1 kappa (κ) type. Preferably the bispecific antibody according to the invention is a trivalent antibody and characterized in comprising a bivalent anti-ROR1 antibody specifically binding to ROR1, and a monovalent antibody specifically binding to CD3. Preferably the monovalent antibody specifically binding to CD3 is a Fab fragment or a single chain Fv fragment. A trivalent antibody is preferred if its said ΔMFI (internalization, %) is between 120% and 100% of the ΔMFI (internalization, %) of the said anti-ROR1 bivalent antibody of human IgG1 kappa (κ) type.

Preferably the bispecific antibody according to the invention is **characterized in that** the bispecific antibody does not internalize in said cell based assay at 37°C during 24 hrs.

Preferably the bispecific antibody according the invention does not internalize in said cell based assay if used in a concentration between 0.1 pM and 200 nM.

A further embodiment of the invention is an antibody according to this invention with an affinity ratio of ROR1 to CD3 of 5000:1 to 5:1, as determined by Kd values using surface plasmon resonance. Such an antibody is favorable because of its stronger binding to malignant cells over T cells. Preferably the Kd values are about 100 nM for the CD3 antibody and about 50 pM to 50 nM for the ROR1 antibody.

Preferably the bispecific antibody according the invention is characterized in comprising
a) the light chain and heavy chain of an antibody specifically binding to one of said targets; and
b) the light chain and heavy chain of an antibody specifically binding to the other one of said targets, wherein the variable domains VL and VH or the constant domains CL and CH1 are replaced by each other.

Preferably the bispecific antibody according to the invention is characterized in
a) consisting of a F(ab)₂ fragment of an anti-ROR1 antibody specifically binding to ROR1, wherein in one arm the variable domains VL and VH or the constant domains CL and CH1 are replaced by each other, with or without Fc part, and
b) consisting of a Fab fragment or a single chain Fv fragment of an antibody specifically binding to CD3 bound to the N-terminus of one VL or VH domain of said antibody F(ab)₂ fragment specifically binding to ROR1.

Preferably the B-CLL cells are used according to the invention in a cell concentration of 15 x 10⁶ cells/mL (primary PBMC from CLL patients) or 2 x 10⁶ cells/mL (ACC-67).

Preferably the antibody according to the invention is further **characterized in that** it binds also specifically to cynomolgus ROR1.

A further embodiment of the invention is a bispecific antibody specifically binding to the two targets human CD3ε (further named also as "CD3") and the extracellular domain of human ROR1 (further named also as "ROR1"), characterized in comprising one Fab fragment of an antibody specifically binding to one of said targets and one Fab fragment of an antibody specifically binding to the other one of said targets.

A further embodiment of the invention is a bispecific antibody specifically binding to the two targets human CD3ε (further named also as "CD3") and the extracellular domain of human ROR1 (further named also as "ROR1"), characterized in comprising one Fab fragment of an antibody specifically binding to one of said targets and two Fab fragment of an antibody specifically binding to the other one of said targets.

Preferably the antibody portion specifically binding to human CD3 is characterized in comprising a variable heavy chain domain VH comprising the CDRs of SEQ ID NO: 12, 13 and 14 as respectively heavy chain CDR1, CDR2 and CDR3 and a variable domain VL comprising the CDRs of SEQ ID NO: 15, 16 and 17 as respectively light chain CDR1, CDR2 and CDR3. Preferably the antibody portion specifically binding to human CD3 is **characterized in that** the variable domains are of SEQ ID NO:10 and 11. Preferably the antibody portion specifically binding to CD3 is characterized in being humanized.

Preferably the antibody portion specifically binding to ROR1 is characterized in comprising a light chain variable domain (VL) comprising as respective variable light chain CDRs the CDRs of SEQ ID NO: 3, 4, 5 and a heavy chain variable domain (VH) comprising as respective variable heavy chain CDRs the CDRs of SEQ ID NO:7, 8, 9. Preferably the antibody portion specifically binding to ROR1 is characterized in comprising as light chain variable domain (VL) a sequence being at least 90% identical to the sequence of SEQ ID NO:2 and as variable heavy chain domain (VH) a sequence being at least 90% identical to the sequence of SEQ ID NO:6, Preferably the antibody portion specifically binding to ROR1 is characterized in comprising as light chain variable domain (VL) the sequence of SEQ ID NO:2 and as variable heavy chain domain (VH) the sequence of SEQ ID NO:6. Preferably the antibody portion specifically binding to ROR1 is characterized in being humanized.

A further embodiment of the invention is a method for the preparation of a bispecific antibody according to the invention comprising the steps of transforming a host cell with one or more vectors comprising nucleic acid molecules encoding the respective antibodies or fragments, culturing the host cell under conditions that allow synthesis of said antibody molecule; and recovering said antibody molecule from said culture.

Preferably the method for the preparation of a bispecific antibody according to the invention comprising the steps of transforming a host cell with one or more vectors comprising nucleic acid molecules encoding
a) a F(ab)₂ fragment of an anti-ROR1 antibody specifically binding to ROR1, wherein in one arm the variable domains VL and VH or the constant domains CL and CH1 are replaced by each other and
b) a Fab fragment of an antibody specifically binding to CD3 bound to the N-terminus of one VL or VH domain of said antibody F(ab)₂ fragment specifically binding to ROR1.
c) culturing the host cell under conditions that allow synthesis of said antibody molecule; and
d) recovering said antibody molecule from said culture.

Preferably the method for the preparation of a bispecific antibody according to the invention is characterized in comprising the steps of
a) transforming a host cell with vectors comprising nucleic acid molecules encoding polypeptides, which form together an antibody molecule which is a F(ab)₂ fragment of an anti-ROR1 antibody specifically binding to ROR1, wherein in one arm the variable domains VL and VH or the constant domains CL and CH1 are replaced by each other and a Fab fragment or a single chain Fv fragment of an antibody specifically binding to CD3 which is linked to the N-terminus of one VL or VH domain of said antibody F(ab)₂ fragment specifically binding to ROR1,
b) culturing the host cell under conditions that allow synthesis of said antibody molecule; and
c) recovering said antibody molecule from said culture.

A further embodiment of the invention is a host cell comprising vectors comprising nucleic acid molecules encoding an antibody according to the invention.

A further embodiment of the invention is a host cell comprising vectors comprising nucleic acid molecules encoding the light chain and heavy chain of an antibody specifically binding to the first target and vectors comprising nucleic acid molecules encoding the light chain and heavy chain of an antibody specifically binding to the second target, wherein the variable domains VL and VH or the constant domains CL and CH1 are replaced by each other.

A further preferred embodiment of the invention is a pharmaceutical composition comprising an antibody according to the invention and a pharmaceutically acceptable excipient.

A further preferred embodiment of the invention is a pharmaceutical composition comprising an antibody according to the invention for use as a medicament. A further preferred embodiment of the invention is an antibody according to the invention or a pharmaceutical composition comprising an antibody according to the invention for use as a medicament in the treatment of ROR1-positive hematological malignancies comprising chronic lymphocytic leukemia (CLL), hairy cell leukemia (HCL), acute lymphoblastic leukemia (ALL), acute myeloid leukemia (AML), chronic myeloid leukemia (CML), mantle cell lymphoma (MCL), marginal zone lymphoma (MZL), diffuse large B cell lymphoma (DLBCL), multiple myeloma (MM) and follicular lymphoma (FL). ROR1 is significantly and uniformly expressed on the cell surface of these various blood cancers. A further embodiment of the invention is an antibody according to the invention or a pharmaceutical composition comprising an antibody according to the invention for use as a medicament in the treatment of leukemias and non-Hodgkin lymphomas expressing ROR1. A preferred embodiment of the invention is an antibody according to the invention or a pharmaceutical composition comprising an antibody according to the invention for use as a medicament in the treatment of chronic lymphocytic leukemia (CLL) of B-cell lineage (B-CLL). B-CLL results from an acquired mutation to the DNA of a single marrow cell that develops into a B lymphocyte. Once the marrow cell undergoes the leukemic change, it multiplies into many cells and overtime crowd out normal cells since CLL cells grow and survive better than normal cells. The result is the uncontrolled growth of CLL cells in the bone marrow, leading to an increase in the number CLL cells in the blood. CLL symptoms usually develop over time with some patients being asymptomatic with only abnormal blood test results (e.g. increase in white blood cells). CLL patients with symptoms experience fatigue, short of breath, anemia, weight loss, decrease in appetite, lymph nodes and spleen enlargement and recurrent infections due to low immunoglobulin levels and decreased neutrophil counts (Leukemia & Lymphoma Society, 2009). A further preferred embodiment of the invention is an antibody according to the invention or a pharmaceutical composition comprising an antibody according to the invention for use as a medicament in the treatment of Multiple Myeloma. A further embodiment of the invention is an antibody according to the invention or a pharmaceutical composition comprising an antibody according to the invention for use as a medicament the treatment of plasma cell disorders like Multiple Myeloma MM or other B-cell disorders expressing ROR1. MM is a B-cell malignancy characterized by a monoclonal expansion and accumulation of abnormal plasma cells in the bone marrow compartment. MM also involves circulating clonal B cells with same IgG gene rearrangement and somatic hypermutation. MM arises from an asymptomatic, premalignant condition called monoclonal gammopathy of unknown significance (MGUS), characterized by low levels of bone marrow plasma cells and a monoclonal protein. MM cells proliferate at low rate. MM results from a progressive occurrence of multiple structural chromosomal changes (e.g. unbalanced translocations). MM involves the mutual interaction of malignant plasma cells and bone marrow microenvironment (e.g. normal bone marrow stromal cells). Clinical signs of active MM include monoclonal antibody spike, plasma cells overcrowding the bone marrow, lytic bone lesions and bone destruction resulting from overstimulation of osteoclasts (Dimopulos & Terpos, Ann Oncol 2010; 21 suppl 7: vii143-150). A further embodiment of the invention is an antibody according to the invention or a pharmaceutical composition comprising an antibody according to the invention for use as a medicament in the treatment of ROR1-positive solid tumors such as human breast cancers (Zhang S, PLoS One 2012; 7(3): e31127) and lung cancers (Yamaguchi T, Cancer Cell 2012; 21(3):348).

A further embodiment of the invention is the use of an antibody according to the invention or the pharmaceutical composition according to the invention for such treatments.

Preferably the antibody according to the invention or the pharmaceutical composition is administered once or twice a week preferably via subcutaneous administration (e.g. preferably in the dose range of 0.25 to 2.5 mg/m²/week or twice a week). Due to superior cytotoxicity activities of the antibody according to the invention it can be administered at least at the same magnitude of clinical dose range (or even lower) as compared to conventional monospecific antibodies or conventional bispecific antibodies that are not T cell bispecifics (i.e. do not bind to CD3 on one arm). It is envisaged that for an antibody according to the invention subcutaneous administration is preferred in the clinical settings (e.g. in the dose range of 100 - 1000 mg/m²/week or twice a week). An antibody according to the invention comprising an Fc part is eliminated with a half-life of about 12 days which allows at least once or twice/week administration Another advantage of the antibody according to the invention is a molecular weight (i.e. approximately 100 - 150 kDa) higher than the kidney filtration size limit (50 -70 kDa) even if the antibody has no Fc part. This molecular weight allows 6 - 72 hrs. elimination half-life and makes subcutaneous administrations once or twice a week possible.

### Detailed Description of the Invention

The term "ROR1" as used herein relates to ROR1 (synonyms: tyrosine-protein kinase transmembrane receptor ROR1, EC=2.7.10.1, neurotrophic tyrosine kinase, receptor-related 1, UniPrtKB Q01973) which is a tyrosine-protein kinase receptor. The extracellular domain of ROR1 consists according to UniProt of amino acids 30 - 406. The term "antibody against ROR1, anti ROR1 antibody or ROR1 Mab" as used herein relates to an antibody specifically binding to human ROR1. The antibody binds specifically to the extracellular domain of ROR1 (amino acids M1-V406 of SEQ ID NO:1). The antibody binds specifically to fragments of the extracellular domain, which are the Ig-like C2-type domain (amino acids Q73-V139 of SEQ ID NO:1), the frizzled domain (amino acids E165-I299 of SEQ ID NO: 1), or the kringle domain (amino acids K312-C391 of SEQ ID NO:1). These fragments are mentioned in WO2005100605. It is further preferred that the antibody binds specifically to the extracellular domain fragment WNISSELNKDSYLTL (SEQ ID NO.18) of ROR1. This fragment is mentioned in Daneshmanesh AH et al., Int. J. Cancer, 123 (2008) 1190-1195.

The term "CD3ε or CD3" as used herein relates to human CD3ε described under UniProt P07766 (CD3E_HUMAN). The term "antibody against CD3, anti CD3 antibody" relates to an antibody binding to CD3ε. Preferably the antibody comprises a variable domain VH comprising the heavy chain CDRs of SEQ ID NO: 12, 13 and 14 as respectively heavy chain CDR1, CDR2 and CDR3 and a variable domain VL comprising the light chain CDRs of SEQ ID NO: 15, 16 and 17 as respectively light chain CDR1, CDR2 and CDR3. Preferably the antibody comprises the variable domains of SEQ ID NO: 10 (VH) and SEQ ID NO:11 (VL). The term "antibody against CD3, anti CD3 antibody" as used herein relates to an antibody specifically binding to CD3.

"Specifically binding to CD3 or ROR1" refer to an antibody that is capable of binding CD3 or ROR1 (the targets) with sufficient affinity such that the antibody is useful as a therapeutic agent in targeting CD3 or ROR1. In some embodiments, the extent of binding of an anti-CD3 or ROR1 antibody to an unrelated, non-CD3 or non-ROR1 protein is about 10-fold preferably >100-fold less than the binding of the antibody to CD3 or ROR1 as measured, e.g., by surface plasmon resonance (SPR) e.g. Biacore®, enzyme-linked immunosorbent (ELISA) or flow cytometry (FACS). Preferably the antibody that binds to CD3 or ROR1 has a dissociation constant (Kd) of 10⁻⁸ M or less, preferably from 10⁻⁸ M to 10⁻¹³ M, preferably from 10⁻⁹ M to 10⁻¹³ M. Preferably the bispecific antibody according to the invention binds to an epitope of ROR1 that is conserved among ROR1 from different species and/or an epitope of CD3 that is conserved among CD3 from different species, preferably among human and cynomolgus. "Bispecific antibody specifically binding to CD3 and ROR1" or "antibody according to the invention" refers to a respective definition for binding to both targets. An antibody specifically binding to ROR1 (or CD3 or ROR1 and CD3) does not bind to other human antigens. Therefore in an ELISA, OD values for such unrelated targets will be equal or lower to that of the limit of detection of the specific assay, preferably equal or lower as 1.5 pM, or equal or lower to OD values of control samples without plate-bound-ROR1 or with untransfected HEK293 cells.

Anti-ROR1 antibodies are analyzed by ELISA for binding to human ROR1 using plate-bound ROR1. For this assay, an amount of plate-bound ROR1 preferably or 1.5 nM and concentration(s) preferably ranging from 1 pM to 200 nM of anti-ROR1 antibody are used. A ROR1 antibody for which its ROR1 binding is at least 20% higher than the OD values of the control samples without plate-bound ROR1 or with untransfected HEK293 cells according to the invention is an anti-ROR1 antibody "binding to human ROR1 in an ELISA assay". An exemplary antibody is an anti-ROR1 antibody, characterized in being of human IgG1 kappa (κ) type comprising as light chain variable domain (VL) the sequence of SEQ ID NO:2 and as variable heavy chain domain (VH) the sequence of SEQ ID NO:6, or the respective CDRs shown in SEQ ID NO: 3, 4, 5, 7, 8, 9 (further named also as "said anti-ROR1 bivalent antibody").

The term "antibody according to the invention which does not internalize" as used herein means a bispecific antibody according to the invention with MFI reduction properties **characterized in that** in a cell based assay at 37°C during 2 hrs., using ROR1-positive B-CLL cells, and used at an antibody concentration of 1 nM, Δ mean fluorescence intensity (ΔMFI), MFI as measured by flow cytometry, reflecting the internalization (%) of the said antibody to ROR1-positive cells is between 120% to 0%, preferably from 100% to 0%, of the ΔMFI (internalization, %) of an anti-ROR1 bivalent antibody of human IgG1 kappa (κ) type comprising as light chain variable domain (VL) the sequence of SEQ ID NO:2 and as variable heavy chain domain (VH) the sequence of SEQ ID NO:6, in the same concentration and experimental conditions. The bispecific antibody according to the invention does not internalize in ROR1-positive B-CLL cells, therefore the binding of the said anti-ROR1 antibody to ROR1-positive B-CLL cells is not reduced about more than 50%, preferably about not more than 30%, when the said antibody is incubated at 37°C for 2 h in such cell based assay as described herein.

The cell line ACC-67 is described in Saltman, D. et al., Leukemia research 14 ( 1990 ) 381-387 and available from Leibniz Institute DSMZ-German Collection of Microorganisms and Cell Cultures, Braunschweig, Germany, in the open collection.

For a therapy using a T cell bispecific antibody comprising an anti-ROR1 antibody, it is preferred that the antibody does not internalize as defined above for facilitating a stable immune synapse between the tumor cell and the T cell and effective T cell-mediated redirected cytotoxicity.

The term "reduction of mean fluorescence intensity" (ΔMFI) reflecting the internalization of the said anti-ROR1 antibody to ROR1-positive cells" or "MFI reduction" as used herein refers to the percentage of MFI reduction as calculated for each ROR1 antibodies relative to the unspecific human IgG control (MFI _{background}) and ROR1 antibodies maintained on ice (MFIₘₐₓ) by using the formula ΔMFI= 100 - 100 X [(MFIₑₓₚₑᵣᵢₘₑₙₜₐₗ - MFI_{background}) / (WFIₘₐₓ - MFI_{background})]. MFIₑₓₚₑᵣᵢₘₑₙₜₐₗ is the MFI measured with said ROR1 antibody after 2h incubation at 37°C. An MFI reduction which is at least 75% blocked and reversed by 10 µM endocytosis inhibitor phenylarsine oxide is caused by antibody internalization while an MFI reduction which is not blocked by phenylarsine oxide is caused by antibody dissociation. Internalizing anti-ROR1 antibodies are known in the state of the art (Baskar et al., Clin. Cancer Res., 14(2): 396-404 (2008)).

The term "between 120% and 0%" means that the value of Δ mean fluorescence intensity (ΔMFI) can have the value of 120%, the value of 0% of ΔMFI of the said anti-ROR1 bivalent antibody of human IgG1 kappa (κ) type and all values between both values.

A ΔMFI (internalization, %) of the said antibody according to the invention equaling to 100% of the ΔMFI (internalization, %) of the said anti-ROR1 bivalent antibody of human IgG1 kappa (κ) type reflects the same internalization than that of the said anti-ROR1 bivalent antibody.

A ΔMFI (internalization, %) of the said antibody according to the invention equaling to 0% of the ΔMFI (internalization, %) of the said anti-ROR1 bivalent antibody of human IgG1 kappa (κ) type reflects 100% less internalization than that of the said anti-ROR1 bivalent antibody.

A ΔMFI (internalization, %) of the said antibody according to the invention equaling between 100% and 0% of the ΔMFI (internalization, %) of the said anti-ROR1 bivalent antibody of human IgG1 kappa (κ) type reflects less internalization, preferably from 0% to 100% less internalization than that of the said anti-ROR1bivalent antibody.

A ΔMFI (internalization, %) of the said antibody according to the invention equaling between 120% and 100% of the ΔMFI (internalization, %) of the said anti-ROR1 bivalent antibody of human IgG1 kappa (κ) type reflects more internalization, preferably from 20% to 0% more internalization than that of the said anti-ROR1 bivalent antibody.

The term "target" as used herein means either ROR1 or CD3.The term "first target and second target" means either CD3 as first target and ROR1 as second target or means ROR1 as first target and CD3 as second target.

The term "antibody" as used herein refers to a a monoclonal antibody or a fragnet thereof. An antibody consists of two pairs of a "light chain" (LC) and a "heavy chain" (HC) (such light chain (LC) /heavy chain pairs are abbreviated herein as LC/HC). The light chains and heavy chains of such antibodies are polypeptides consisting of several domains. Each heavy chain comprises a heavy chain variable region (abbreviated herein as HCVR or VH) and a heavy chain constant region. Each light chain comprises a light chain variable domain VL and a light chain constant domain CL. The variable domains VH and VL can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The "constant domains" of the heavy chain and of the light chain are not involved directly in binding of an antibody to a target, but exhibit various effector functions.

The term "devoid of the Fc domain" as used herein means that the bispecific antibodies according to the invention do not comprise a CH2, CH3 or CH4 domain; i.e. the constant heavy chain consists solely of one or more CH1 domains.

The term "antibody" includes e.g. mouse antibodies, human antibodies, chimeric antibodies, humanized antibodies and genetically engineered antibodies (variant or mutant antibodies) as long as their characteristic properties are retained. Especially preferred are human or humanized antibodies, especially as recombinant human or humanized antibodies.

The term "comprising" in regard to the bispecific antibody as used herein means that the bispecific antibody comprises as CD3 and ROR1 binders only those binders mentioned. Therefore a bispecific antibody according the invention comprising a monovalent anti-ROR1 antibody specifically binding to ROR1, and a monovalent antibody specifically binding to CD3 has in regard to CD3 and ROR1 binding only one binding valence for CD3 and only one valence for ROR1and is therefore bivalent. A bispecific antibody according the invention comprising a bivalent anti-ROR1 antibody specifically binding to ROR1, and a monovalent antibody specifically binding to CD3 has in regard to ROR1 binding two binding valences and in regard to CD3 binding one valence and is therefore trivalent. Preferably the monovalent antibody specifically binding to CD3 is covalently linked at its C-terminus to the N-terminus of one variable chain of the antibody specifically binding to ROR1.

As used herein, "Fab fragment" refers to an antibody fragment comprising a light chain fragment comprising a VL domain and a constant domain of a light chain (CL), and a VH domain and the first constant domain (CH1) of a heavy chain. Due to the exchange of either the variable regions or the constant regions in one of the anti-ROR1 antibody Fab fragments (ROR1 Fab), said ROR1 Fab is also referred to as "cross-Fab fragment" or "xFab fragment" or "crossover Fab fragment". Two different chain compositions of a crossover Fab molecule are possible and comprised in the bispecific antibodies of the invention: On the one hand, the variable regions of the Fab heavy and light chain are exchanged, i.e. the crossover Fab molecule comprises a peptide chain composed of the light chain variable region (VL) and the heavy chain constant region (CH1), and a peptide chain composed of the heavy chain variable region (VH) and the light chain constant region (CL). This crossover Fab molecule is also referred to as CrossFab (VLVH). On the other hand, when the constant regions of the Fab heavy and light chain are exchanged, the crossover Fab molecule comprises a peptide chain composed of the heavy chain variable region (VH) and the light chain constant region (CL), and a peptide chain composed of the light chain variable region (VL) and the heavy chain constant region (CH1). This crossover Fab molecule is also referred to as CrossFab (CLCH1). The term "Fab fragment" also includes parts or all of the hinge region, like Fab' fragment. As used herein, "F(ab)₂ fragment" refers to a bivalent monospecific antibody fragment without an Fc part. Preferably a F(ab)₂ fragment is linked at the C-terminus by disulphide bond(s) in the hinge region and usually such a "F(ab)₂ fragment" is a F(ab')₂ fragment.

In one embodiment the Fab fragment of the antibody specifically binding to CD3 (CD3 Fab) is connected via a peptide linker to form a single chain Fv fragment (CD3 scFv). By "connected" is meant that the Fab fragments are linked by peptide bonds, either directly or via one or more peptide linker. Also the CD3 Fab or the CD3 scFv, respectively is linked at its C-terminus in such a manner to the N-terminus of a VH or VL region of the antibody specifically binding to ROR1.

The term "peptide linker" as used within the invention denotes a peptide with amino acid sequences, which is preferably of synthetic origin. These peptide linkers according to invention are used to connect one of the Fab fragments to the C-or N-terminus of the other Fab fragment to form a multispecific antibody according to the invention. Preferably said peptide linkers are peptides with an amino acid sequence with a length of at least 5 amino acids, preferably with a length of 5 to 100, more preferably of 10 to 50 amino acids. In one embodiment said peptide linker is (GxS)n or (GxS)nGm with G = glycine, S = serine, and (x = 3, n= 3, 4, 5 or 6, and m= 0, 1, 2 or 3) or (x = 4,n= 2, 3, 4 or 5 and m= 0, 1, 2 or 3), preferably x = 4 and n= 2 or 3, more preferably with x = 4, n= 2. Additionally, linkers may comprise (a portion of) an immunoglobulin hinge region. In one embodiment said peptide linker is (G₄S)₂ (SEQ ID: NO 19).

There are five types of mammalian antibody heavy chains denoted by the Greek letters: α, δ, ε, γ, and µ (Janeway CA, Jr et al (2001). Immunobiology. 5th ed., Garland Publishing). The type of heavy chain present defines the class of antibody; these chains are found in IgA, IgD, IgE, IgG, and IgM antibodies, respectively (Rhoades RA, Pflanzer RG (2002). Human Physiology, 4th ed., Thomson Learning). Distinct heavy chains differ in size and composition; α and γ contain approximately 450 amino acids, while µ and ε have approximately 550 amino acids. Each heavy chain has two regions, the constant region and the variable region. The constant region is identical in all antibodies of the same isotype, but differs in antibodies of different isotype. Heavy chains γ, α and δ have a constant region composed of three constant domains CH1, CH2, and CH3 (in a line) , and a hinge region for added flexibility (Woof J, Burton D Nat Rev Immunol 4 (2004) 89-99); heavy chains µ and ε have a constant region composed of four constant domains CH1, CH2, CH3, and CH4 (Janeway CA, Jr et al (2001). Immunobiology. 5th ed., Garland Publishing). The variable region of the heavy chain differs in antibodies produced by different B cells, but is the same for all antibodies produced by a single B cell or B cell clone. The variable region of each heavy chain is approximately 110 amino acids long and is composed of a single antibody domain. In mammals there are only two types of light chain, which are called lambda (λ) and kappa (κ). A light chain has two successive domains: one constant domain CL and one variable domain VL. The approximate length of a light chain is 211 to 217 amino acids.

A bispecific antibody according to the invention, which comprises a Fc part, can be of any class (e.g. IgA, IgD, IgE, IgG, and IgM, preferably IgG or IgE), or subclass (e.g., IgG1, IgG2, IgG3, IgG4, IgA1 and IgA2, preferably IgG1), whereby both antibodies, from which the bivalent bispecific antibody according to the invention is derived, have an Fc part of the same subclass( e.g. IgG1, IgG4 and the like, preferably IgG1), preferably of the same allotype (e.g. Caucasian).

A "Fc part of an antibody" is a term well known to the skilled artisan and defined on the basis of papain cleavage of antibodies. The antibodies according to the invention, which comprise an Fc part, contain as Fc part, preferably a Fc part derived from human origin and preferably all other parts of the human constant regions. The Fc part of an antibody is directly involved in complement activation, C1q binding, C3 activation and Fc receptor binding. While the influence of an antibody on the complement system is dependent on certain conditions, binding to C1q is caused by defined binding sites in the Fc part. Such binding sites are known in the state of the art and described e.g. by Lukas, TJ., et al., J. Immunol. 127 (1981) 2555-2560; Brunhouse, R., and Cebra, J.J., MoI. Immunol. 16 (1979) 907-917; Burton, D.R., et al., Nature 288 (1980) 338-344; Thommesen, J.E., et al., MoI. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat, see below). Antibodies of subclass IgG1, IgG2 and IgG3 usually show complement activation, C1q binding and C3 activation, whereas IgG4 do not activate the complement system, do not bind C1q and do not activate C3. Preferably the Fc part is a human Fc part.

The constant heavy chain of an antibody according to the invention is preferably of human IgG1type and the constant light chain is preferably of human lambda (λ) or kappa (κ) type, preferably of human kappa (κ) type.

The terms "monoclonal antibody" or "monoclonal antibody composition" as used herein refer to a preparation of antibody molecules of a single amino acid composition.

The term "chimeric antibody" refers to an antibody comprising a variable region, i.e., binding region, from one source or species and at least a portion of a constant region derived from a different source or species, usually prepared by recombinant DNA techniques. Chimeric antibodies comprising a murine variable region and a human constant region are preferred. Other preferred forms of "chimeric antibodies" encompassed by the present invention are those in which the constant region has been modified or changed from that of the original antibody to generate the properties according to the invention, especially in regard to C1q binding and/or Fc receptor (FcR) binding. Such chimeric antibodies are also referred to as "class-switched antibodies". Chimeric antibodies are the product of expressed immunoglobulin genes comprising DNA segments encoding immunoglobulin variable regions and DNA segments encoding immunoglobulin constant regions. Methods for producing chimeric antibodies involve conventional recombinant DNA and gene transfection techniques are well known in the art. See, e.g., Morrison, S.L., et al., Proc. Natl. Acad. Sci. USA 81 (1984) 6851-6855; US Patent Nos. 5,202,238 and 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the targets noted above for chimeric antibodies. Other forms of "humanized antibodies" encompassed by the present invention are those in which the constant region has been additionally modified or changed from that of the original antibody to generate the properties according to the invention, especially in regard to C1q binding and/or Fc receptor (FcR) binding.

The term "human antibody", as used herein, is intended to include antibodies having variable and constant regions derived from human germ line immunoglobulin sequences. Human antibodies are well-known in the state of the art (van Dijk, M.A., and van de Winkel, J.G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production. Transfer of the human germ-line immunoglobulin gene array in such germ-line mutant mice will result in the production of human antibodies upon target challenge (see, e.g., Jakobovits, A., et al., Proc. Natl. Acad. Sci. USA 90 (1993) 2551-2555; Jakobovits, A., et al., Nature 362 ( 1993) 255-258; Bruggemann, M., et al., Year Immunol. 7 (1993) 33-40). Human antibodies can also be produced in phage display libraries (Hoogenboom, H.R., and Winter, G., J. MoI. Biol. 227 (1992) 381-388; Marks, J.D., et al., J. MoI. Biol. 222 (1991) 581-597). The techniques of Cole et al. and Boerner et al. are also available for the preparation of human monoclonal antibodies (Cole et al., Monoclonal Antibodies and Cancer Therapy, Alan R. Liss, p. 77 (1985); and Boerner, P., et al., J. Immunol. 147 (1991) 86-95).

The term "recombinant human antibody", as used herein, is intended to include all human antibodies that are prepared, expressed, created or isolated by recombinant means, such as antibodies isolated from a host cell such as a NSO or CHO cell or from an animal (e.g. a mouse) that is transgenic for human immunoglobulin genes or antibodies expressed using a recombinant expression vector transfected into a host cell. Such recombinant human antibodies have variable and constant regions in a rearranged form. The recombinant human antibodies according to the invention have been subjected to in vivo somatic hypermutation. Thus, the amino acid sequences of the VH and VL regions of the recombinant antibodies are sequences that, while derived from and related to human germ line VH and VL sequences, may not naturally exist within the human antibody germ line repertoire in vivo.

The "variable domain" (variable domain of a light chain (VL), variable region of a heavy chain (VH)) as used herein denotes each of the pair of light and heavy chains which is involved directly in binding the antibody to the target. The domains of variable human light and heavy chains have the same general structure and each domain comprises four framework (FR) regions whose sequences are widely conserved, connected by three "hypervariable regions" (or complementarity determining regions, CDRs). The framework regions adopt a β-sheet conformation and the CDRs may form loops connecting the β-sheet structure. The CDRs in each chain are held in their three-dimensional structure by the framework regions and form together with the CDRs from the other chain the target binding site. The antibody heavy and light chain CDR3 regions play a particularly important role in the binding specificity/affinity of the antibodies according to the invention and therefore provide a further object of the invention.

The terms "hypervariable region" or "target-binding portion of an antibody" when used herein refer to the amino acid residues of an antibody which are responsible for target-binding. The hypervariable region comprises amino acid residues from the "complementarity determining regions" or "CDRs". "Framework" or "FR" regions are those variable domain regions other than the hypervariable region residues as herein defined. Therefore, the light and heavy chains of an antibody comprise from N- to C-terminus the domains FR1, CDR1, FR2, CDR2, FR3, CDR3, and FR4. CDRs on each chain are separated by such framework amino acids. Especially, CDR3 of the heavy chain is the region which contributes most to target binding. CDR and FR regions are determined according to the standard definition of Kabat et al., Sequences of Proteins of Immunological Interest, 5th ed., Public Health Service, National Institutes of Health, Bethesda, MD (1991).

The term "target" or "target molecule" as used herein are used interchangeable and refer to human ROR1 and human CD3ε.

The term "epitope" includes any polypeptide determinant capable of specific binding to an antibody. In certain embodiments, epitope determinant include chemically active surface groupings of molecules such as amino acids, sugar side chains, phosphoryl, or sulfonyl, and, in certain embodiments, may have specific three dimensional structural characteristics, and or specific charge characteristics. An epitope is a region of a target that is bound by an antibody.

In general there are two vectors encoding the light chain and heavy chain of said antibody specifically binding to the first target, and further two vectors encoding the light chain and heavy chain of said antibody specifically binding to the second target. One of the two vectors is encoding the respective light chain and the other of the two vectors is encoding the respective heavy chain. However in an alternative method for the preparation of a bispecific antibody according to the invention, only one first vector encoding the light chain and heavy chain of the antibody specifically binding to the first target and only one second vector encoding the light chain and heavy chain of the antibody specifically binding to the second target can be used for transforming the host cell.

The term "nucleic acid or nucleic acid molecule", as used herein, is intended to include DNA molecules and RNA molecules. A nucleic acid molecule may be single-stranded or double-stranded, but preferably is double-stranded DNA.

As used herein, the expressions "cell," "cell line," and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included. Where distinct designations are intended, it will be clear from the context.

The term "transformation" as used herein refers to process of transfer of a vectors/nucleic acid into a host cell. If cells without formidable cell wall barriers are used as host cells, transfection is carried out e.g. by the calcium phosphate precipitation method as described by Graham and Van der Eh, Virology 52 (1978) 546ff. However, other methods for introducing DNA into cells such as by nuclear injection or by protoplast fusion may also be used. If prokaryotic cells or cells which contain substantial cell wall constructions are used, e.g. one method of transfection is calcium treatment using calcium chloride as described by Cohen SN, et al, PNAS 1972, 69 (8): 2110-2114.

Recombinant production of antibodies using transformation is well-known in the state of the art and described, for example, in the review articles of Makrides, S. C, Protein Expr. Purif. 17 (1999) 183-202; Geisse, S., et al., Protein Expr. Purif. 8 (1996) 271-282; Kaufman, RJ., MoI. Biotechnol. 16 (2000) 151-161; Werner, R.G., et al., Arzneimittelforschung 48 (1998) 870-880 as well as in US6331415 and US4816567.

As used herein, "expression" refers to the process by which a nucleic acid is transcribed into mRNA and/or to the process by which the transcribed mRNA (also referred to as transcript) is subsequently being translated into peptides, polypeptides, or proteins. The transcripts and the encoded polypeptides are collectively referred to as gene product. If the polynucleotide is derived from genomic DNA, expression in a eukaryotic cell may include splicing of the mRNA.

A "vector" is a nucleic acid molecule, in particular self-replicating, which transfers an inserted nucleic acid molecule into and/or between host cells. The term includes vectors that function primarily for insertion of DNA or RNA into a cell (e.g., chromosomal integration), replication of vectors that function primarily for the replication of DNA or RNA, and expression vectors that function for transcription and/or translation of the DNA or RNA. Also included are vectors that provide more than one of the functions as described.

An "expression vector" is a polynucleotide which, when introduced into an appropriate host cell, can be transcribed and translated into a polypeptide. An "expression system" usually refers to a suitable host cell comprised of an expression vector that can function to yield a desired expression product.

The bispecific antibodies according to the invention are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression, nucleic acids encoding light and heavy chains or fragments thereof are inserted into expression vectors by standard methods. Expression is performed in appropriate prokaryotic or eukaryotic host cells like CHO cells, NSO cells, SP2/0 cells, HEK293 cells, COS cells, yeast, or E.coli cells, and the antibody is recovered from the cells (supernatant or cells after lysis). The bispecific antibodies may be present in whole cells, in a cell lysate, or in a partially purified or substantially pure form. Purification is performed in order to eliminate other cellular components or other contaminants, e.g. other cellular nucleic acids or proteins, by standard techniques, including alkaline/SDS treatment, column chromatography and others well known in the art. See Ausubel, F., et al., ed., Current Protocols in Molecular Biology, Greene Publishing and Wiley Interscience, New York (1987).

Expression in NS0 cells is described by, e.g., Barnes, L.M., et al., Cytotechnology 32 (2000) 109-123; and Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; and Norderhaug, L., et al., J. Immunol. Methods 204 (1997) 77-87. A preferred transient expression system (HEK293) is described by Schlaeger, E.- J., and Christensen, K., in Cytotechnology 30 (1999) 71-83 and by Schlaeger, E.-J., in J. Immunol. Methods 194 (1996) 191-199.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the

DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The bispecific antibodies are suitably separated from the culture medium by conventional immunoglobulin purification procedures such as, for example, protein A-Sepharose, hydroxylapatite chromatography, gel electrophoresis, dialysis, or affinity chromatography. DNA or RNA encoding the monoclonal antibodies is readily isolated and sequenced using conventional procedures. The hybridoma cells can serve as a source of such DNA and RNA. Once isolated, the DNA may be inserted into expression vectors, which are then transfected into host cells such as HEK293 cells, CHO cells, or myeloma cells that do not otherwise produce immunoglobulin protein, to obtain the synthesis of recombinant monoclonal antibodies in the host cells.

Amino acid sequence variants (or mutants) of the bispecific antibody are prepared by introducing appropriate nucleotide changes into the antibody DNA, or by nucleotide synthesis. Such modifications can be performed, however, only in a very limited range, e.g. as described above. For example, the modifications do not alter the above mentioned antibody characteristics such as the IgG isotype and target binding, but may improve the yield of the recombinant production, protein stability or facilitate the purification.

T cell bispecific (TCB) binders have very high concentration/tumor-cell-receptor-occupancy dependent potency in cell killing (e.g. EC₅₀ in in vitro cell killing assays in the sub- or low picomolar range; Dreier et al. Int J Cancer 2002), T-cell bispecific binder (TCB) are given at much lower doses than conventional monospecific antibodies. For example, blinatumomab (CD19xCD3) is given at a continuous intravenous dose of 5 to 15 µg/m²/day (i.e. only 0.35 to 0.105 mg/m²/week) for treatment of acute lymphocytic leukemia or 60 µg/m²/day for treatment of Non Hodgkin Lymphoma, and the serum concentrations at these doses are in the range of 0.5 to 4 ng/ml (Klinger et al., Blood 2012; Topp et al., J Clin Oncol 2011; Goebeler et al. Ann Oncol 2011). Because low doses of TCB can exert high efficacy in patients, it is envisaged that for an antibody according to the invention subcutaneous administration is possible and preferred in the clinical settings (preferably in the dose range of 0.25 to 2.5 mg/m²/week or twice a week). Even at these low concentrations/doses/receptor occupancies, TCB can cause considerable adverse events (Klinger et al., Blood 2012).

In principle it is possible to produce bispecific antibodies against CD3 and ROR1 in all formats known in the state of the art. A wide variety of recombinant bispecific antibody formats have been developed in the recent past, e.g. by fusion of, e.g. an IgG antibody format and single chain domains (see e.g. Kontermann RE, mAbs 4:2, (2012) 1-16). Bispecific antibodies wherein the variable domains VL and VH or the constant domains CL and CH1 are replaced by each other are described in WO2009080251 and WO2009080252. Antibody formats and formats of bispecific and multispecific antibodies are also pepbodies (WO200244215), Novel Antigen Receptor ("NAR") (WO2003014161), diabody-diabody dimers "TandAbs" (WO2003048209), polyalkylene oxide-modified scFv (US7150872), humanized rabbit antibodies (WO2005016950), synthetic immunoglobulin domains (WO2006072620), covalent diabodies (WO2006113665), flexibodies (WO2003025018), domain antibodies, dAb (WO2004058822), vaccibody (WO2004076489), antibodies with new world primate framework (WO2007019620), antibody-drug conjugate with cleavable linkers (WO2009117531), IgG4 antibodies with hinge region removed (WO2010063785), bispecific antibodies with IgG4 like CH3 domains (WO2008119353), camelid Antibodies (US6838254), nanobodies (US7655759), CAT diabodies (US5837242), bispecific scFv2 directed against target antigen and CD3 (US7235641), ), sIgA plAntibodies (US6303341), minibodies (US5837821), IgNAR (US2009148438), antibodies with modified hinge and Fc regions (US2008227958, US20080181890), trifunctional antibodies (US5273743), triomabs (US6551592), troybodies (US6294654).

However an antibody according to the invention that is devoid of an Fc part has a safety advantage because of the lack of possibility for inducing Fc-mediated infusion reactions. Because of the strong potency of a bispecific antibody according to the invention in killing of target cells in vitro (e.g. in the subnanomolar and even picomolar range) and the fact that ROR1 is also expressed on normal cells (e.g. normal human adipocytes), it is preferred that the bispecific antibody can be eliminated rapidly from the circulation after being administrated. A T cell bispecific devoid of an Fc part according to the invention has therefore a safety advantage and is eliminated rapidly from the circulation in case of toxicity.

An antibody according to the invention which has as two or three F(ab) fragments a molecular weight of about 100 kDa and 143 kDa respectively, i.e. bigger than the kidney filtration size limit but without an Fc, gives the TCB a half-lifeof 6 to 72 hours enabling once or twice a week sc administration but being shorter in half life than a Fc containing antibody (10 to 14 days), has safety advantages over a TCB with Fc and does not show Fc-related infusion reactions.

A bispecific trivalent antibody according to the invention has advantages on the potency, predictability for efficacy and safety.

An antibody according to the invention with bivalency to ROR1 and monovalency to CD3 favors binding to the tumor target ROR1 on malignant cells over CD3ε on T cells in circulation and avoids CD3 sink, thus increasing drug exposure in the tumor.

The following examples, sequence listing and figures are provided to aid the understanding of the present invention, the true scope of which is set forth in the appended claims. It is understood that modifications can be made in the procedures set forth without departing from the spirit of the invention.

### Materials & general methods

General information regarding the nucleotide sequences of human immunoglobulins light and heavy chains is given in: Kabat, E. A., et al., Sequences of Proteins of Immunological Interest, 5th ed., Public Health Service, National Institutes of Health, Bethesda, MD (1991). Amino acids of antibody chains are numbered and referred to according to EU numbering (Edelman, G.M., et al., Proc. Natl. Acad. Sci. USA 63 (1969) 78-85; Kabat, E.A., et al., Sequences of Proteins of Immunological Interest, 5th ed., Public Health Service, National Institutes of Health, Bethesda, MD, (1991)).

### Recombinant DNA techniques

Standard methods are used to manipulate DNA as described in Sambrook, J. et al., Molecular cloning: A laboratory manual; Cold Spring Harbor Laboratory Press, Cold Spring Harbor, New York, 1989. The molecular biological reagents are used according to the manufacturer's instructions.

### Gene synthesis

Desired gene segments are prepared from oligonucleotides made by chemical synthesis. The 600 - 1800 bp long gene segments, which are flanked by singular restriction endonuclease cleavage sites, are assembled by annealing and ligation of oligonucleotides including PCR amplification and subsequently cloned via the indicated restriction sites e.g. Kpnl/ Sad or Ascl/Pacl into a pPCRScript (Stratagene) based pGA4 cloning vector. The DNA sequences of the subcloned gene fragments are confirmed by DNA sequencing. Gene synthesis fragments are ordered according to given specifications at Geneart (Regensburg, Germany).

### DNA sequence determination

DNA sequences are determined by double strand sequencing performed at MediGenomix GmbH (Martinsried, Germany) or Sequiserve GmbH (Vaterstetten, Germany).

### DNA and protein sequence analysis and sequence data management

The GCG's (Genetics Computer Group, Madison, Wisconsin) software package version 10.2 and Infomax's Vector NT1 Advance suite version 8.0 is used for sequence creation, mapping, analysis, annotation and illustration.

### Expression vectors

For the expression of the described antibodies variants of expression plasmids for transient expression (e.g. in HEK293 EBNA or HEK293-F) cells based either on a cDNA organization with a CMV-Intron A promoter or on a genomic organization with a CMV promoter are applied. Beside the antibody expression cassette the vectors contain an origin of replication which allows replication of this plasmid in E. coli, and- a β-lactamase gene which confers ampicillin resistance in E. coli. The transcription unit of the antibody gene is composed of the following elements:
- unique restriction site(s) at the 5' end - the immediate early enhancer and promoter from the human cytomegalovirus,
- followed by the Intron A sequence in the case of the cDNA organization,
- a 5'-untranslated region of a human antibody gene,
- a immunoglobulin heavy chain signal sequence,
- the human antibody chain (wildtype or with domain exchange) either as cDNA or as genomic organization with an the immunoglobulin exon-intron organization
- a 3' untranslated region with a polyadenylation signal sequence, and
- unique restriction site(s) at the 3' end.

The fusion genes comprising the described antibody chains as described below are generated by PCR and/or gene synthesis and assembled with known recombinant methods and techniques by connection of the according nucleic acid segments e.g. using unique restriction sites in the respective vectors. The subcloned nucleic acid sequences are verified by DNA sequencing. For transient transfections larger quantities of the plasmids are prepared by plasmid preparation from transformed E. coli cultures (Nucleobond AX, Macherey-Nagel).

### Cell culture techniques

Standard cell culture techniques are used as described in Current Protocols in Cell Biology (2000), Bonifacino, J. S., Dasso, M., Harford, J.B., Lippincott-Schwartz, J. and Yamada, K.M. (eds.), John Wiley & Sons, Inc.

### Transient expression in HEK293 cells

Bispecific antibodies are expressed by transient co-transfection of the respective expression plasmids in adherently growing HEK293-EBNA or in HEK293-F cells growing in suspension as described below.

### a) Transient transfections in HEK293-EBNA system

Bispecific antibodies are expressed by transient co-transfection of the respective expression plasmids (e.g. encoding the heavy and modified heavy chain, as well as the corresponding light and modified light chain) in adherently growing HEK293-EBNA cells (human embryonic kidney cell line 293 expressing Epstein-Barr-Virus nuclear target; American type culture collection deposit number ATCC # CRL- 10852, Lot. 959 218) cultivated in DMEM (Dulbecco's modified Eagle's medium, Gibco) supplemented with 10% Ultra Low IgG FCS (fetal calf serum, Gibco), 2 mM L-Glutamine (Gibco), and 250 µg/ml Geneticin (Gibco). For transfection FuGENE™ 6 Transfection Reagent (Roche Molecular Biochemicals) is used in a ratio of FuGENE™ reagent (µl) to DNA (µg) of 4:1 (ranging from 3:1 to 6:1).

Proteins are expressed from the respective plasmids using a molar ratio of (modified and wildtype) light chain and heavy chain encoding plasmids of 1:1 (equimolar) ranging from 1:2 to 2:1, respectively. Cells are feeded at day 3 with L-Glutamine ad 4 mM, Glucose [Sigma] and NAA [Gibco]. Bispecific antibody containing cell culture supernatants are harvested from day 5 to 11 after transfection by centrifugation and stored at -200C. General information regarding the recombinant expression of human immunoglobulins in e.g. HEK293 cells is given in: Meissner, P. et al., Biotechnol. Bioeng. 75 (2001) 197-203.

### b) Transient transfections in HEK293-F system

Bispecific antibodies are generated by transient transfection of the respective plasmids (e.g. encoding the heavy and modified heavy chain, as well as the corresponding light and modified light chain) using the HEK293-F system (Invitrogen) according to the manufacturer's instruction. Briefly, HEK293-F cells (Invitrogen) growing in suspension either in a shake flask or in a stirred fermenter in serumfree FreeStyle 293 expression medium (Invitrogen) are transfected with a mix of the four expression plasmids and 293fectin or fectin (Invitrogen). For 2 L shake flask (Corning) HEK293-F cells are seeded at a density of 1.0 x 10⁶ cells/mL in 600 mL and incubated at 120 rpm, 8% CO2. The day after the cells are transfected at a cell density of ca. 1.5 x 10⁶ cells/mL with ca. 42 mL mix of A) 20 mL Opti-MEM (Invitrogen) with 600 µg total plasmid DNA (1 µg/mL) encoding the heavy or modified heavy chain, respectively and the corresponding light chain in an equimolar ratio and B) 20 ml Opti-MEM + 1.2 mL 293 fectin or fectin (2 µl/mL). According to the glucose consumption glucose solution is added during the course of the fermentation. The supernatant containing the secreted antibody is harvested after 5-10 days and antibodies are either directly purified from the supernatant or the supernatant is frozen and stored.

### Protein determination

The protein concentration of purified antibodies and derivatives is determined by determining the optical density (OD) at 280 nm, using the molar extinction coefficient calculated on the basis of the amino acid sequence according to Pace et al., Protein Science, 1995, 4, 2411-1423.

### Antibody concentration determination in supernatants

The concentration of antibodies and derivatives in cell culture supernatants is estimated by immunoprecipitation with Protein A Agarose-beads (Roche). 60 µL Protein A Agarose beads are washed three times in TBS-NP40 (50 mM Tris, pH 7.5, 150 mM NaCl, 1% Nonidet-P40).

Subsequently, 1 -15 mL cell culture supernatant is applied to the Protein A Agarose beads pre-equilibrated in TBS-NP40. After incubation for at 1 h at room temperature the beads are washed on an Ultrafree-MC-filter column (Amicon] once with 0.5 mL TBS-NP40, twice with 0.5 mL 2x phosphate buffered saline (2xPBS, Roche) and briefly four times with 0.5 mL 100 mM Na-citrate pH 5,0. Bound antibody is eluted by addition of 35 µl NuPAGE® LDS Sample Buffer (Invitrogen). Half of the sample is combined with NuPAGE® Sample Reducing Agent or left unreduced, respectively, and heated for 10 min at 70°C. Consequently, 5-30 µl are applied to an 4-12% NuPAGE® Bis-Tris SDS-PAGE (Invitrogen) (with MOPS buffer for non-reduced SDS-PAGE and MES buffer with NuPAGE® Antioxidant running buffer additive (Invitrogen) for reduced SDS-PAGE) and stained with Coomassie Blue.

The concentration of antibodies and derivatives in cell culture supernatants is quantitatively measured by affinity HPLC chromatography. Briefly, cell culture supernatants containing antibodies and derivatives that bind to Protein A are applied to an Applied Biosystems Poros A/20 column in 200 mM KH₂PO₄, 100 mM sodium citrate, pH 7.4 and eluted from the matrix with 200 mM NaCl, 100 mM citric acid, pH 2,5 on an Agilent HPLC 1100 system. The eluted protein is quantified by UV absorbance and integration of peak areas. A purified standard IgG1 antibody served as a standard.

Alternatively, the concentration of antibodies and derivatives in cell culture supernatants is measured by Sandwich-IgG-ELISA. Briefly, StreptaWell High Bind Strepatavidin A-96 well microtiter plates (Roche) are coated with 100 µL/well biotinylated anti-human IgG capture molecule F(ab')2<h-Fcγ> BI (Dianova) at 0.1 µg/mL for 1 h at room temperature or alternatively over night at 4°C and subsequently washed three times with 200 µL/well PBS, 0.05% Tween® (PBST, Sigma). 100 µL/well of a dilution series in PBS (Sigma) of the respective antibody containing cell culture supernatants is added to the wells and incubated for 1-2 h on a micro titerplate shaker at room temperature. The wells are washed three times with 200 µL/well PBST and bound antibody is detected with 100 µl F(ab')2<hFcγ>POD (Dianova) at 0.1 µg/mL as detection antibody for 1-2 h on a microtiterplate shaker at room temperature. Unbound detection antibody is washed away three times with 200 µL/well PBST and the bound detection antibody is detected by addition of 100 µL ABTS/well. Determination of absorbance is performed on a Tecan Fluor Spectrometer at a measurement wavelength of 405 nm (reference wavelength 492 nm).

### Protein purification

Proteins are purified from filtered cell culture supernatants referring to standard protocols. In brief, antibodies are applied to a Protein A Sepharose column (GE healthcare) and washed with PBS. Elution of antibodies is achieved at pH 2.8 followed by immediate neutralization of the sample.

Aggregated protein is separated from monomeric antibodies by size exclusion chromatography (Superdex 200, GE Healthcare) in PBS or in 20 mM Histidine, 150 mM NaCl pH 6.0. Monomeric antibody fractions are pooled, concentrated if required using e.g. a MILLIPORE Amicon Ultra (30 MWCO) centrifugal concentrator, frozen and stored at -20°C. Part of the samples are provided for subsequent protein analytics and analytical characterization e.g. by SDS-PAGE, size exclusion chromatography or mass spectrometry.

### SDS-PAGE

The NuPAGE® Pre-Cast gel system (Invitrogen) is used according to the manufacturer's instruction. In particular, 10% or 4-12% NuPAGE® Novex® Bis-TRIS Pre-Cast gels (pH 6.4) and a NuPAGE® MES (reduced gels, with NuPAGE® Antioxidant running buffer additive) or MOPS (non-reduced gels) running buffer is used.

### Analytical size exclusion chromatography

Size exclusion chromatography for the determination of the aggregation and oligomeric state of antibodies is performed by HPLC chromatography. Briefly, Protein A purified antibodies are applied to a Tosoh TSKgel G3000SW column in 300 mM NaCl, 50 mM KH₂PO₄/K₂HPO₄, pH 7.5 on an Agilent HPLC 1100 system or to a Superdex 200 column (GE Healthcare) in 2 x PBS on a Dionex HPLC-System. The eluted protein is quantified by UV absorbance and integration of peak areas. BioRad Gel Filtration Standard 151-1901 served as a standard.

### Mass spectrometry

The total deglycosylated mass of crossover antibodies is determined and confirmed via electrospray ionization mass spectrometry (ESI-MS). Briefly, 100 µg purified antibodies are deglycosylated with 50 mil N-Glycosidase F (PNGaseF, ProZyme) in 100 mM KH₂PO₄/K₂HPO₄, pH 7 at 37°C for 12-24 h at a protein concentration of up to 2 mg/ml and subsequently desalted via HPLC on a Sephadex G25 column (GE Healthcare). The mass of the respective heavy and light chains is determined by ESI-MS after deglycosylation and reduction. In brief, 50 µg antibody in 115 µl are incubated with 60 µl IM TCEP and 50 µl 8 M Guanidine-hydrochloride subsequently desalted. The total mass and the mass of the reduced heavy and light chains is determined via ESI-MS on a Q-Star Elite MS system equipped with a NanoMate® source.

### Examples

### Example 1- Generation of anti-ROR1 antibodies

The protein sequences of the VH and VL regions for an ROR1 antibody of SEQ ID NOs: 2-9 are described in WO2012/075158.

Briefly, oliogonucleotides encoding the above sequences are joined together via PCR to synthesize cDNAs encoding the VH are VL sequences, respectively, of the anti-ROR1 antibody.

### Example 1A. Recombinant, soluble, human ROR1 extracellular domain

Recombinant, soluble, human ROR1 extracellular domain is produced as a fusion protein to the C-terminus of human IgG1 Fc ("ROR1-ECD"). Briefly, using splice-overlap-extension PCR, a cDNA encoding the fusion protein is synthesized. The cDNA encodes, from N- to C-terminus, a fusion protein consisting of a secretory leader sequence, hinge region, CH2 and CH3 domains of human IgG1, a flexible linker with the sequence Gly-Gly-Gly-Gly-Ser-Gly-Gly-Gly-Gly-Ser, (SEQ ID NO:19) and the extracellular domains of human ROR1 (amino acid residues 24 - 403). The cDNA is subcloned into a mammalian expression vector and the recombinant fusion protein is produced and purified using the same methods as for human IgG1 antibodies described below in Example 2. The template used for PRC-amplifying the human ROR1 extracellular domain-encoding cDNA portion is a human full-length cDNA clone EN1031_D08 Ror1 gene (Origene Technologies, Rockville, MD).

A biotinylated variant of the same human Fc-ROR1 ECD fusion ("ROR1-ECD-biot") is produced as described above using the same procedures with the following modifications. A DNA sequence encoding an Avi-His tag is added, via PCR amplification, in frame downstream at the 3' end of the first PCR product described above. This new, second PCR product is then subcloned into the mammalian expression vector and then co-transfected in mammalian cells together with a vector for expression of BirA enzyme for in vivo biotinylation of the Avi tag. During cell culture for production of ROR1-ECD-biot, cell culture medium is supplemented with biotin to allow for proper biotinylation. The remaining production and purification steps are performed as indicated above for ROR1-ECD.

### Example 1B. Recombinant cells expressing human ROR1 on their surface

Recombinant cells expressing human ROR1 on their surface ("HEK293-ROR1 cells") are generated as described in example 1A. The amplification product is cloned into an E. coli expression vector, comprising human cytomegalovirus (CMV) immediate early enhancer/promoter, a polyhistidine (6xHis), and a neomycin resistance gene, linearized and transfected into human embryonic kidney 293 (HEK293) cells. These cells are selected which express human ROR1 on their surface high expressing stable clones are chosen by fluorescence-activated cell sorting analysis.

### Example 1C. Human B-CLL cell line expressing ROR1 on their surface

Human B-CLL cell line EHEB is acquired from Leibniz Institute DSMZ-German Collection of Microorganisms and Cell Cultures (ACC-67). EHEB cells are grown in RPMI supplemented with 10% fetal bovine serum. ROR1 expression on EHEB cell lines is confirmed by flow cytometry using fluorochrome-conjugated anti-human ROR1 antibodies (BD Biosciences).

### Example 1D. Obtaining anti-ROR1 antibodies via immunization

Anti-ROR1 antibodies are generated by immunization of rats with ROR1 ECD. Briefly, Sprague-Dawley rats are immunized subcutaneously with keyhole limpet hemocyanin-conjugated ROR1 ECD (amino acids 5-54; NP_001183) using TiterMax® adjuvant (Sigma). Keyhole limpet hemocyanin conjugation is performed with a lysine residue using Imject mcKLHV (Pierce). Due to the high sequence homology between human and mouse ROR1 proteins, rats are preferred for antibody production. B cells are harvested from immunized spleens and fused to P3-X63.Ag8 myeloma cells using a standard polyethylene glycol fusion protocol (Goding 1996; Monoclonal antibodies: principles and practice. 3rd ed. Academic Press). Hybridomas are cultured in 80% Iscove's modified Dulbecco's medium supplemented with 10% fetal clone I, 4 mmol/L L-glutamine, 10% cloning factor and also including penicillin, streptomycin and 1× sodium hypoxanthine, aminopterin, and thymidine. ELISA testing is performed to detect binding of hybridoma culture supernatants to ROR1. Positive ROR1-binding hybridomas are further screened by flow cytometry for cell-based binding to ROR1 transfectants (HEK293-ROR1 cells). Chosen hybridomas undergo two rounds of limiting dilution cloning and are further expanded for purification. In addition, antibodies from those same chosen hybridomas are converted to chimeric antibodies with human constant regions by standard methods. Briefly, cDNAs encoding the heavy and light chain variable regions are amplified bz RT-PCR out of mRNA from the hybridomas and then joined in frame with cDNAs coding the heavy constant region of human IgG1 and the human kappa light chain constant region, respectively. These cDNAs are cloned into mammalian transient expression vectors and plasmid DNA is produced in E. coli and purified for transfection. HEK293 cells are transfected by a standard transfection method (calcium phosphate-based transfection) and 7 days later IgG1 antibodies are purified from culture supernatants by affinity chromatography on a Protein A column followed by isolation of the monomeric antibody fraction via size exclusion chromatography.

### Example 1E. Obtaining anti-ROR1 antibodies out of an in vitro, recombinant library

### Example 1E1. Construction of Generic Fab-Libraries

Generic antibody libraries in the Fab-format are constructed on the basis of human germline genes using the following V-domain pairings: Vk3_20 kappa light chain with VH3_23 heavy chain for the DP47-3 library and Vk1_17 kappa light chain with VH1_69 heavy chain for the DP88-3 library. Both libraries are randomized in CDR3 of the light chain (L3) and CDR3 of the heavy chain (H3) and are assembled from 3 fragments per library by splicing by overlapping extension (SOE) PCR. Fragment 1 comprises the 5' end of the antibody gene including randomized L3, fragment 2 is a central constant fragment spanning from L3 to H3, whereas fragment 3 comprises randomized H3 and the 3' portion of the antibody gene. The following primer combinations are used to generate library fragments for DP47-3 library: fragment 1 (LMB3-LibL1b_new), fragment 2 (MS63-MS64), fragment 3 (Lib2H-fdseqlong) (see table 1 of WO2012020038). The following primer combinations are used to generate library fragments for the DP88-3 library: fragment 1 (LMB3-RJH_LIB3), fragment 2 (RJH31-RJH32) and fragment 3 (LIB88_2-fdseqlong). See tables 3 and 4 of WO2012020038.

The PCR protocol for the production of library fragments includes: 5 min of initial denaturation at 94°C; 25 cycles of 1 min at 94°C, 1 min at 58° C, and 1 min at 72°C; and terminal elongation for 10 min at 72°C. For assembly PCR, equimolar ratios of the 3 fragments are used as template. The assembly PCR protocol includes: 3 min of initial denaturation at 94°C; and 5 cycles of 30 seconds at 94°C, 1 min at 58° C, and 2 min at 72°C. At this stage, primers complementary to sequence outside fragments 1-3 are added and an additional 20 cycles are performed prior to a terminal elongation for 10 min at 72°C. After assembly of sufficient amounts of full length randomized Fab constructs, the Fab constructs are digested with NcoI/NotI for the DP47-3 library and with NcoI/NheI for the DP88-3 library alongside with similarly treated acceptor phagemid vector. For the DP47-3 library, 22.8 µg of Fab library is ligated with 16.2 µg of phagemid vector. For the DP88-3 library, 30.6 µg of Fab library is ligated with 30.6 µg of phagemid vector.

Purified ligations are used for 68 transformations for the DP47-3 library and 64 transformations for the DP88-3 library, respectively, to obtain final DP47-3 and DP88-3 libraries. Phagemid particles displaying the Fab libraries are rescued and purified by PEG/NaCl purification to be used for selection of anti-ROR1 Fab clones.

### Example 1E2. Selection of Anti-ROR1 Fab Clones

Selections are carried out against ROR1-ECD-biot. The antigen is biotinylated in vivo upon expression. Selections are carried out in solution according to the following protocol: (i) binding of ^{∼}10¹² phagemid particles of library DP88-3 and 100 nM ROR1-ECD-biot for 0.5 hours in a total volume of 1 ml; (ii) capture of ROR1-ECD-biot and attached phage by the addition of 5.4×10⁷ streptavidin-coated magnetic beads for 10 min; (iii) washing of beads using 5×1 ml PBS/Tween®20 and 5×1 ml PBS; (iv) elution of phage particles by the addition of 1 mL 100 mM TEA (triethylamine) for 10 min and neutralization by the addition of 500 µL 1M Tris/HCl pH 7.4; and (v) re-infection of log-phase *E. coli* TG1 cells (Zymo Research), infection with helper phage VCSM13 (Stratagene) and subsequent PEG/NaCl precipitation of phagemid particles to be used in subsequent selection rounds.

Selections are carried out over 3 rounds using constant ROR1-ECD-biot concentrations at 100 nM. In round 2, capture of antigen:phage complexes is performed on neutravidin plates instead of streptavidin beads. Specific binders are identified by ELISA as follows using: 100 µl of 100 nM ROR1-ECD-biot is coated in each well of neutravidin plates.

Fab-containing bacterial supernatants are added and binding Fabs are detected via their Flag-tags by using an anti-Flag/HRP secondary antibody. Once identified, anti-ROR1 ECD clones are bacterially expressed in a 0.5 litre culture volume, affinity purified and further characterized by SPR-analysis using a BIACORE® instrument.

### Example IF. ROR1 binding assays: surface plasmon resonance

To measure binding affinities of ROR1 antibody to immobilized ROR1, surface plasmon resonance measurements are performed on a Biacore® 3000 instrument (Pharmacia Biosensor). The receptor ROR1 (ROR1-ECD) is coupled to the sensor chip at a level of 400 resonance units using the amine coupling protocol as provided by manufacturer. Alternative ROR1-ECD-biot is coupled to a streptavidin-sensor chip, also at a level of 400 resonance units, using the protocol as provided by the manufacturer. In all experiments, flow cell 1 is used as the reference cell. Sensorgrams are recorded for Fab solutions ranging in concentrations from 0.1 pM to 200 nM. Nonlinear regression analysis is used to calculate kinetic constants and binding constants simultaneously with the use of the manufacturer's software. Fab clones with monovalent binding affinities to ROR1-ECD of ≤ 100 nM are converted into IgGs by standard methods. Briefly, cDNAs encoding the heavy and light chain variable regions are joined in frame with cDNAs coding the heavy constant region of human IgG1 and the human kappa light chain constant region, respectively. These cDNAs are cloned into mammalian transient expression vectors and plasmid DNA is produced in E. coli and purified for transfection. HEK293 cells are transfected by a standard transfection method (calcium phosphate-based transfection) and 7 days later IgG1 antibodies are purified from culture supernatants by affinity chromatography on a Protein A column followed by isolation of the monomeric antibody fraction via size exclusion chromatography.

### Example 1H. Binding to ROR1 on HEK293-ROR1 cells or plate-bound-ROR1 (Flow cytometry and ELISA)

Anti-ROR1 antibodies coming either from the immunization approach and/or from the screening of the recombinant in vitro library described above are analyzed by flow cytometry for binding to human ROR1 on HEK293-ROR1 cells. Briefly, cultured cells are harvested, counted and cell viability is evaluated using the Trypan Blue exclusion method. Viable cells are then adjusted to 2 x 10⁶ cells per ml in BSA-containing FACS Stain Buffer (BD Biosciences). 90 µl of this cell suspension are further aliquoted per well into a round-bottom 96-well plate. 10 µl of the anti-ROR1 antibodies or corresponding IgG control are added to the cell-containing wells to obtain final concentrations of 0.1 pM to 200 nM. All constructs and control IgG are used at the same molarity. After incubation for 30 min at 4°C, the cells are centrifuged (5 min, 350 x g), washed with 150 µl/well FACS Stain Buffer (BD Biosciences), resuspended and incubated for an additional 30 min at 4°C with 12 µl/well fluorochrome-conjugated AffiniPure F(ab')₂ Fragment goat anti-human IgG Fcγ Fragment Specific (Jackson Immuno Research Lab; working solution: 1:20). Cells are then washed with Stain Buffer (BD Biosciences) 120 µl/wel and pelleted down by centrifugation at 350 x g for 5 min. A second washing step is performed using FACS Stain Buffer 150 µl/well. The samples are resuspended in 200 µl/well FACS Stain Buffer and acquired and analyzed using an LSR II flow cytometer with FACSDiva® software (BD Biosciences). The mean fluorescence intensity (MFI) is plotted as a function of anti-ROR1 antibody concentration to obtain the binding curve and to calculate the effective antibody concentration to reach 50% of maximal binding (EC₅₀). Anti-ROR1 antibodies that bind to ROR1 on cells as judged from this assay are selected for the next screening step, namely the internalization assay (step (Example 1I) below).

The properties of antibodies that show binding to human ROR1 on HEK293-ROR1 cells are confirmed using a conventional ELISA method. Briefly, immunosorb 96-well plates are coated with 1.5 µg/mL of GST-ROR1-ECD, washed with PBS + 1% Tween (PBS-T), and blocked with PBS-T plus 1% serum albumin. ROR1-coated plates are incubated with hybridoma culture supernatants for 2 h at room temperature, washed 5 times with PBS-T, and incubated with peroxidase-conjugated goat-anti-rat IgG. Following incubation with secondary antibody, plates are washed, incubated with 3,3,5,5-tetramethylbenzidine substrate, and stopped with an equal volume of 1 mol/L H₂SO₄.

### Example 1I. Internalization of anti-ROR1 antibody on EHEB B-CLL cell line or primary PBMC from CLL patients (Flow cytometry)

Anti-ROR1 antibodies selected in step (Example 1H) above are further tested in the internalization assay. Using a 96-well U-bottom plate, 3 x 10⁶ (15 x 10⁶ cells/mL) cryopreserved PBMC from untreated CLL patients are first blocked with 500 nM unspecific polyclonal human IgG at room temperature for 20 min, then stained with 5 nM biotinylated anti-ROR1 antibodies on ice for 1 h. After washing three times with FACS Stain Buffer (BD Biosciences) to remove unbound antibody, the cells are either left on ice or incubated at 37°C for 15 min, 30 min, 1 h, 2 h, and 24 h to facilitate internalization. In addition, the cells are incubated at 37°C for 2 h and/or 24 h in the presence of 10 µM phenylarsine oxide (Sigma-Aldrich) to inhibit internalization. Subsequently, the cells are washed once with FACS Stain Buffer and incubated with PE-streptavidin on ice for 30 min. After three final washes with FACS Stain Buffer, the mean fluorescence intensity (MFI) of the cells is measured using a LSR II flow cytometer with FACSDiva® software (BD Biosciences) and FlowJo analytical software.

Instead of PBMC from untreated CLL patients the EHEB B-CLL cell line (Leibniz Institute DSMZ - German Collection of Microorganisms and Cell Cultures) can be used in the internalization assay at a concentration of 2 x 10⁶ cells/mL.

MFI reduction can represent antibody internalization, antibody dissociation or a combination of both. The percentage of MFI reduction is calculated for each ROR1 antibodies relative to the unspecific human IgG control (MFI _{background}) and ROR1 antibodies maintained on ice (MFIₘₐₓ) by using the formula ΔMFI= 100 - 100 X [(MFIₑₓₚₑᵣᵢₘₑₙₜₐₗ - MFI_{background}) / (MFIₘₐₓ - MFI_{background})]· An MFI reduction which is blocked by endocytosis inhibitor phenylarsine oxide suggests antibody internalization while an MFI reduction which is not blocked by phenylarsine oxide reflects antibody dissociation. Internalizing anti-ROR1 antibodies are known in the state of the art (Baskar et al., Clin. Cancer Res., 14(2): 396-404 (2008)).

For antibody-based therapies such as T cell bispecifics, it is important that the antibody or antibody fragment specific to the tumor target do not internalize, or slowly internalize, or slightly internalize for facilitating a stable immune synapse between the tumor cell and the T cell and effective T cell-mediated redirected cytotoxicity. Thus, anti-ROR1 antibodies selected in step (Example 1H) above which does not internalize or slowly internalize or slightly internalize are selected for the next step (Example 2) below, namely the production of anti-ROR1 antibodies.

### Example 2 - Production of anti-ROR1 antibodies

Selected antibodies that are derived from immunization are in a rat-human chimeric format and are then humanized to be able to apply them for therapy. In that case, standard antibody humanization methods are applied by transferring the complementarity-determining regions of those rat variable regions into human antibody variable region frameworks. Additional mutations are introduced into the variable regions, if necessary, to recover binding to ROR1 as compared to the chimeric, parental antibody.

For the production of the antibody, the cells are co-transfected with two plasmids, (one for expression of the heavy chain of the antibody and another for expression of the light chain of the antibody), at a ratio of 1:1, respectively. Cells are grown as adherent monolayer cultures in T flasks using DMEM culture medium supplemented with 10% FCS, and are transfected when they are between 50 and 80% confluent. For the transfection of a T75 flask, 8 million cells are seeded 24 hours before transfection in 14 ml DMEM culture medium supplemented with FCS (at 10% V/V final), 250 µg/ml neomycin, and cells are placed at 37°C in an incubator with a 5% CO₂ atmosphere overnight. For each T75 flask to be transfected, a solution of DNA, CaCl₂ and water is prepared by mixing 47 µg total plasmid vector DNA divided equally between the light and heavy chain expression vectors, 235 µl of a 1M CaCl₂ solution, and adding water to a final volume of 469 µl. To this solution, 469 µl of a 50 mM HEPES, 280 mM NaCl, 1.5 mM Na₂ HPO₄ solution at pH 7.05 are added, mixed immediately for 10 sec and left to stand at room temperature for 20 sec. The suspension is diluted with 12 ml of DMEM supplemented with 2% FCS, and added to the T75 in place of the existing medium. The cells are incubated at 37°C., 5% CO₂ for about 17 to 20 hours, then medium is replaced with 12 ml DMEM, 10% FCS. The conditioned culture medium is harvested 5 to 7 days post-transfection centrifuged for 5 min at 1200 rpm, followed by a second centrifugation for 10 min at 4000 rpm and kept at 4°C.

The secreted antibodies are purified by Protein A affinity chromatography, followed by cation exchange chromatography and a final size exclusion chromatographic step on a Superdex® 200 column (Amersham Pharmacia) exchanging the buffer to phosphate buffer saline and collecting the pure monomeric IgG1 antibodies. Antibody concentration is estimated using a spectrophotometer from the absorbance at 280 nm. The antibodies were formulated in a 25 mM potassium phosphate, 125 mM sodium chloride, 100 mM glycine solution of pH 6.7.

### Example 3 - Generation of anti-ROR1/anti-CD3 T cell bispecific F(ab)₂ antibodies

### Example 3A. Generation of anti-CD3 antibodies

The following protein sequences of the VH and VL regions are used to generate human and cynomolgus monkey cross reactive CD3ε antibodies as described in WO2007/042261.
**H2C_VH (SEQ ID NO:7):** 
EVQLVESGGGLVQPGGSLKLSCAASGFTFNKYAMNWVRQAPGKGLEWVARIRSKYNNY
ATYYADSVKDRFTISRDDSKNTAYLQMNNLKTEDTAVYYCVRHGNFGNSYISYWAYWG
QGTLVTVSS
 **H2C_VL** (SEQ ID NO:8)

QTVVTQEPSLTVSPGGTVTLTCGSSTGAVTSGYYPNWVQQKPGQAPRGLIGGTKFLAPGT
PARFSGSLLGGKAALTLSGVQPEDEAEYYCALWYSNRWVFGGGTKLTVL

Briefly, oligonucleotides encoding the above sequences are joined together via PCR to synthesize cDNAs encoding the VH are VL sequences, respectively, of the anti-CD3 antibody.

### Example 3B. Generation of anti-ROR1/anti-CD3 T cell bispecific 1+1 format (i.e. bispecific (Fab) x (Fab) antibody monovalent for ROR1 and monovalent for CD3) without Fc

An anti-ROR1/anti-CD3 T cell bispecific antibody that is devoid of an Fc part would have a safety advantage because of the lack of possibility for inducing Fc-mediated infusion reactions. In addition, because of the strong potency of T cell bispecific antibodies in killing of target cells in vitro (e.g. subnanomlolar and even picomolar range), it is desirable that for T cell bispecific antibodies specific for a tumor target that is also expressed on normal cells, such as ROR1 on normal human adipocytes, they could be eliminated rapidly from the circulation after being administrated. A T cell bispecific devoid of an Fc would have a safety advantage and would be eliminated more rapidly from the circulation in case of toxicity.

Anti-ROR1/anti-CD3 T cell bispecific of the 1+1 format (i.e. bispecific (Fab)x(Fab) antibody monovalent for ROR1 and monovalent for CD3 without Fc are produced for the human or humanized anti-ROR1 antibodies selected after step (Example 1). cDNAs encoding the full Fabs (heavy chain VH and CH1 domains plus light chain VL and CL domains) of the corresponding anti-ROR1 IgG1 antibodies, as described in Example 1, as well as the anti-CD3 VH and VL cDNAs describe in Example 3A, are used as the starting materials. For each bispecific antibody, four protein chains are involved comprising the heavy and light chains of the corresponding anti-ROR1 antibody and the heavy and light chains of the anti-CD3 antibody described above, respectively, without any Fc regions.

Briefly, each bispecific antibody is produced by simultaneous cotransfection of three mammalian expression vectors encoding, respectively: a) the full light chain cDNA of the corresponding ROR1 antibody, b) a fusion cDNA generated by standard molecular biology methods, such as splice-overlap-extension PCR, encoding a fusion protein made of (in N- to C-terminal order) secretory leader sequence, Fab (VH followed by CH1 domains) of the corresponding anti-ROR1 antibody described above, a flexible glycine(Gly)-serine(Ser) linker with the sequence Gly-Gly-Gly- Gly-Ser-Gly-Gly- Gly- Gly-Ser, the VH of the anti-CD3 antibody described above and the constant kappa domain of a human light chain cDNA, d) a fusion cDNA generated by standard molecular biology methods, such as splice-overlap-extension PC, encoding a fusion protein made of (in N- to C-terminal order) secretory leader sequence, VL of the anti-CD3 antibody described above, constant CH1 domain of a human IgG1 cDNA. Co-transfection of mammalian cells and antibody production and purification using the methods described above for production of human or humanized IgG1 antibodies (see Example 2), with one modification: for purification of antibodies, the first capture step is not done using ProteinA, but instead is done using an affinity chromatography column packed with a resin binding to human kappa light chain constant region, such as KappaSelect (GE Healthcare Life Sciences). In addition, a disulfide can be included to increase the stability and yields as well as additional residues forming ionic bridges and increasing the heterodimerization yields (EP 1870459A1).

### Example 3C. Generation of anti-ROR1/anti-CD3 T cell bispecific 2+1 format (i.e. bispecific (Fab)₂ x (Fab) antibody bivalent for ROR1 and monovalent for CD3) without Fc

An anti-ROR1/anti-CD3 T cell bispecific antibody with a 2+1 format i.e. bispecific (Fab)₂ x (Fab) antibody that is bivalent for ROR1 and monovalent for CD3 would have advantages on potency, predictability for efficacy and safety because it would preferentially bind to the tumor target ROR1 and avoid CD3 antibody sink, thus higher probability for drug exposure in the tumor.

Anti -ROR1/anti-CD3 T cell bispecific of the 2+1 format (i.e. bispecific (Fab)₂ x (Fab) antibody bivalent for ROR1 and monovalent for CD3 without Fc are produced for the human or humanized anti-ROR1 antibodies selected after step (Example 1). cDNAs encoding the full Fabs (heavy chain VH and CH1 domains plus light chain VL and CL domains)of the corresponding anti-ROR1 IgG1 antibodies, as described in Example 1, as well as the anti-CD3 VH and VL cDNAs describe in Example 3A, are used as the starting materials. For each bispecific antibody, four protein chains are involved comprising the heavy and light chains of the corresponding anti-ROR1 antibody and the heavy and light chains of the anti-CD3 antibody described above, respectively, without any Fc regions.

Briefly, each bispecific antibody is produced by simultaneous cotransfection of three mammalian expression vectors encoding, respectively: a) the full light chain cDNA of the corresponding ROR1 antibody, b) a fusion cDNA generated by standard molecular biology methods, such as splice-overlap-extension PCR, encoding a fusion protein made of (in N- to C-terminal order) secretory leader sequence, Fab (VH followed by CH1 domains) of the corresponding anti-ROR1 antibody described above, a flexible glycine(Gly)-serine(Ser) linker with the sequence Gly-Gly-Gly- Gly-Ser-Gly-Gly- Gly- Gly-Ser, Fab (VH followed by CH1 domains) of the corresponding anti-ROR1 antibody described above, a flexible glycine(Gly)-serine(Ser) linker with the sequence Gly-Gly- Gly- Gly-Ser-Gly-Gly- Gly- Gly-Ser, the VH of the anti-CD3 antibody described above and the constant kappa domain of a human light chain cDNA, d) a fusion cDNA generated by standard molecular biology methods, such as splice-overlap-extension PC, encoding a fusion protein made of (in N- to C-terminal order) secretory leader sequence, VL of the anti-CD3 antibody described above, constant CH1 domain of a human IgG1 cDNA. Co-transfection of mammalian cells and antibody production and purification using the methods described above for production of human or humanized IgG1 antibodies (see Example 2), with one modification: for purification of antibodies, the first capture step is not done using ProteinA, but instead is done using an affinity chromatography column packed with a resin binding to human kappa light chain constant region, such as KappaSelect (GE Healthcare Life Sciences). In addition, a disulfide can be included to increase the stability and yields as well as additional residues forming ionic bridges and increasing the heterodimerization yields (EP 1870459A1).

### Example 4 - Simultaneous binding of anti-ROR1/anti-CD3 T cell bispecific antibodies to ROR1 and CD3 (Surface plasmon resonance)

The binding properties to ROR1 and CD3 of bispecific anti-ROR1/anti-CD3 T cell bispecific antibodies generated in Example 3 are analyzed by surface plasmon resonance (SPR) technology using a Biacore® T100 instrument (Biacore AB) with HBS-EP as running buffer (0.01 M HEPES pH 7.4, 0.15 M NaCl, 3 mM EDTA, 0.005% Surfactant P20, Biacore). This system is well established for the study of molecule interactions. It allows a continuous real-time monitoring of ligand/analyte bindings and thus the determination of association rate constants (ka), dissociation rate constants (kd), and equilibrium constants (KD) in various assay settings. SPR-technology is based on the measurement of the refractive index close to the surface of a gold coated biosensor chip. Changes in the refractive index indicate mass changes on the surface caused by the interaction of immobilized ligand with analyte injected in solution. If molecules bind to immobilized ligand on the surface the mass increases, in case of dissociation the mass decreases.

Capturing anti-His tag antibody is immobilized on the surface of a CM5 biosensorchip using amine-coupling chemistry. Flow cells are activated with a 1:1 mixture of 0.1 M N-hydroxysuccinimide and 0.1 M 3-(N,N-dimethylamino)propyl-N-ethylcarbodiimide at a flow rate of 5 µl/min anti-human IgG antibody is injected in sodium acetate, pH 5.0 at 10 µg/ml, which resulted in a surface density of approximately 12000 resonance units (RU). A reference control flow cell is treated in the same way but with vehicle buffers only instead of the capturing antibody. Surfaces are blocked with an injection of 1 M ethanolamine/HCl pH 8.5. The anti-ROR1/anti-CD3 T cell bispecific antibodies are diluted in HBS-P and injected at a flow rate of 5 µl/min. The contact time (association phase) is 1 min for the antibodies at a concentration between 1 and 100 nM for the ROR1-ECD binding and 1 and 200 nM for the CD3 interaction. ROR1-ECD is injected at increasing concentrations of 3.125, 6.25, 12.5, 25, 50 and 100 nM, CD3 at concentrations of 0.21, 0.62, 1.85, 5.6, 16.7, 50, 100 and 200 nM. The contact time (association phase) is 3 min, the dissociation time (washing with running buffer) 5 min for both molecules at a flowrate of 30 µl/min. All interactions are performed at 25°C (standard temperature). The regeneration solution of 3 M Magnesium chloride is injected for 60 s at 5 µl/min flow to remove any non-covalently bound protein after each binding cycle. Signals are detected at a rate of one signal per second. Samples are injected at increasing concentrations. SPR graphs showing the rate of signal (i.e. resonance unit) plotted against contact time are determined.

### Example 5 - Binding of anti-ROR1/anti-CD3 T cell bispecific antibodies to ROR1 B-CLL cells or CD3 on T cells (Flow cytometry)

Anti-ROR1/anti-CD3 T cell bispecific antibodies generated in Example 3 are also analyzed by flow cytometry for their binding properties to human ROR1 expressed on EHEB B-CLL cells or human CD3 expressed on human leukemic T cells Jurkat (ATCC). Briefly, cultured cells are harvested, counted and cell viability is evaluated using the Trypan Blue exclusion method. Viable cells are then adjusted to 2 x 10⁶ cells per ml in PBS containing 0.1% BSA. 90 µl of this cell suspension are further aliquoted per well into a round-bottom 96-well plate. 10 µl of the T cell bispecific antibody or corresponding IgG control are added to the cell-containing wells to obtain final concentrations of 0.1 pM to 200 nM. Anti-ROR1/anti-CD3 T cell bispecific antibodies and control IgG are used at the same molarity. After incubation for 30 min at 4°C, cells are centrifuged (5 min, 350 x g), washed with 150 µl/well BSA-containing FACS Stain Buffer (BD Biosciences), resuspended and incubated for an additional 30 min at 4°C with 12 µl/well fluorochrome-conjugated anti-His antibody (Lucerna) for detection of the T cell bispecific antibody. Cells are then washed by addition of 120 µl/wel FACS Stain Buffer and centrifugation at 350 x g for 5 min. A second washing step is performed with 150 µl/well FACS Stain Buffer. The samples are resuspended in 200 µl/well FACS Stain Buffer, acquired and analyzed using an LSR II flow cytometer with FACSDiva® software (BD Biosciences). Binding of the anti-ROR1/anti-CD3 T cell bispecific antibodies to B-CLL cells and T cells are evaluated and the mean fluorescence intensity is determined gated on either ROR1-expressing EHEB B-CLL cells or CD3-expressing Jurkat T cells and plotted in histograms or dot plots.

### Example 6. - Internalization of anti-ROR1/anti-CD3 T cell bispecific antibodies on EHEB B-CLL cell line or primary PBMC from CLL patients (Flow cytometry).

Anti-ROR1/anti-CD3 T cell bispecific antibodies selected in step (Example 5) above are further tested in the internalization assay. Using a 96-well U-bottom plate, 3 x 10⁶ (15 x 10⁶/mL) cryopreserved PBMC from untreated CLL patients or 2 x10⁶cells/mL of EHEB B-CLL cell line are first blocked with 500 nM unspecific polyclonal human IgG at room temperature for 20 min, then stained with 1 nM biotinylated anti-ROR1/anti-CD3 T cell bispecific antibodies on ice for 1 h. After washing three times with FACS Stain Buffer (BD Biosciences) to remove unbound antibody, the cells are either left on ice or incubated at 37°C for 15 min, 30 min, 1 h, 2 h, and 24 h to facilitate internalization. In addition, the cells are incubated at 37°C for 2 h and/or 24 h in the presence of 10 µM phenylarsine oxide (Sigma-Aldrich) to inhibit internalization. Subsequently, the cells are washed once with FACS Stain Buffer and incubated with PE-streptavidin on ice for 30 min. After three final washes with FACS Stain Buffer, the mean fluorescence intensity (MFI) of the cells is measured using a LSR II flow cytometer with FACSDiva® software (BD Biosciences) and FlowJo analytical software.

The term "reduction of mean fluorescence intensity" (ΔMFI) reflecting the internalization of the said anti-ROR1 antibody into ROR1-positive cells" or "MFI reduction" as used herein refers to the percentage of MFI reduction as calculated for each ROR1 antibodies relative to the unspecific human IgG control (MFI background) and ROR1 antibodies maintained on ice (MFIₘₐₓ) by using the formula ΔMFI= 100 - 100 X [(MFIₑₓₚₑᵣᵢₘₑₙₜₐₗ - MFI_{background}) / (MFIₘₐₓ - MFI_{background})]. MFIₑₓₚₑᵣᵢₘₑₙₜₐₗ is the MFI measured with said ROR1 antibody after 2h incubation at 37°C. MFI reduction can represent antibody internalization, antibody dissociation or a combination of both.. An MFI reduction which is blocked by endocytosis inhibitor phenylarsine oxide suggests antibody internalization while an MFI reduction which is not blocked by phenylarsine oxide reflects antibody dissociation. Internalizing anti-ROR1 antibodies are known in the state of the art (Baskar et al., Clin. Cancer Res., 14(2): 396-404 (2008)).

The internalization rate of anti-ROR1/anti-CD3 T cell antibodies is then compared to that of anti-ROR1bivalent antibodies.

For antibody-based therapies such as T cell bispecifics, it is important that the antibody or antibody fragment specific to the tumor target do not internalize, or slowly internalize, or slightly internalize for facilitating a stable immune synapse between the tumor cell and the T cell and effective T cell-mediated redirected cytotoxicity.

### Example 7 - Activation of T cells upon engagement of anti-ROR1/anti-CD3 T cell bispecific antibodies (Flow cytometry)

Anti-ROR1/anti-CD3 T cell bispecific antibodies generated in Example 3 are also analyzed by flow cytometry for their potential to induce T cell activation by evaluating the surface expression of the early activation marker CD69, or the late activation marker CD25 on CD4⁺ and CD8⁺ T cells in the presence or absence of human ROR1-expressing B-CLL cells. Briefly, ROR1-expressing EHEB B-CLL cells are harvested with Cell Dissociation buffer, counted and cell viability is verified using Trypan Blue. Viable B-CLL cells are adjusted to 0.2 x 10⁶ cells/mL in complete RPMI-1640 medium, 100 µl of this cell suspension per well is pipetted into a round-bottom 96-well plate. 50 µl of the T cell bispecific constructs are added to the B-CLL cells-containing wells to obtain a final concentration of 1 nM. The 96-well plate is set aside and kept at 37°C, 5% CO₂ until further manipulations.

PBMC are isolated from fresh blood using density gradient centrifugation using Cell Preparation Tubes with Sodium citrate (Vacutainer CPT tubes, BD Biosciences). Total human T cells are then isolated using the Pan T Cell Isolation Kit II (Miltenyi Biotec), according to the manufacturer's instructions. Human total T cells (effector) are then adjusted to 2 x 10⁶ cells per ml in complete RPMI-1640 medium. 50 µl of this cell suspension is added per well in the assay plate containing already ROR1-expressing B-CLL cells to obtain a final E:T ratio of 5:1. To test whether the T cell bispecific constructs are able to activate T cells only in the presence of ROR1-expressing B-CLL tumor target cells, wells containing final concentration(s) in the range of 0.1 pM to 200 nM of the respective bispecific molecules with effector cells but without B-CLL tumor target cells are also included. After incubation for five days at 37°C, 5% CO₂, cells are pelleted down by centrifugation (5 min, 350 x g) and washed twice with 150 µl/well of FACS Stain Buffer (BD Biosciences). Surface staining of the effector cells with selected fluorochrome-conjugated antibodies against human CD4, CD8, CD69 or CD25 (BD Biosciences) is performed at 4°C for 30 min, protected from light, in FACS Stain Buffer (BD Biosciences) according to the manufacturer's protocol. Cells are washed twice with 150 µl/well FACS Stain Buffer, resuspended in 200 µl/well FACS Stain Buffer, and acquired and analyzed using a LSRII flow cytometer complemented with FACSDiva® software (BD Biosciences). The expression of CD69 and CD25 activation markers are determined by measuring the mean fluorescence intensity gated on CD4⁺ and CD8⁺ T cell populations as represented in histograms or dot plots.

### Example 8 - Proliferation of T cells upon engagement of anti-ROR1/anti-CD3 T cell bispecific antibodies (CFSE dilution)

Anti-ROR1/anti-CD3 T cell bispecific antibodies generated in Example 3 are also analyzed by flow cytometry for their potential to induce proliferation of CD8⁺ or CD4⁺ T cells in the presence or absence of human ROR1-expressing B-CLL cells. Briefly, ROR1-expressing EHEB B-CLL cells are harvested with Cell Dissociation buffer, counted and looked for viability using Trypan Blue. Viable B-CLL cells are adjusted to 0.2 x 10⁶ cells per ml in complete RPMI medium, 100 µl of this cell suspension are pipetted per well into a round-bottom 96-well plate. 50 µl of the T cell bispecific constructs are added to the B-CLL-containing wells to obtain final concentration(s) in the range of 0.1 pM to 200 nM. The well plate is set aside and kept at 37°C, 5% CO₂.

PBMC are isolated from fresh blood using density gradient centrifugation using Cell Preparation Tubes with Sodium citrate (Vacutainer CPT tubes, BD Biosciences). Total human T cells are then isolated using the Pan T Cell Isolation Kit II (Miltenyi Biotec), according to the manufacturer's instructions. The total T cells are then adjusted to 1 million cells per ml in pre-warm RPMI without serum (37°C) and stained with 1 µM CFSE at room temperature for 6 min, protected from light. The staining volume is then doubled by addition of RPMI-1640 medium supplemented with 10% FCS and 1% GlutaMax to stop CFSE staining. After incubation at room temperature for further 20 min, the cells are washed three times with pre-warmed serum-containing medium to remove remaining CFSE. CFSE-stained total T cells (effector) are then adjusted to 2 x 10⁶ cells/mL in complete RPMI-1640 medium. 50 µl of this cell suspension is added per well in the assay plate already containing ROR1-expressing EHEB B-CLL cells to obtain a final E:T ratio of 5:1. To test whether the T cell bispecific constructs are able to activate T cells only in the presence of ROR1-expressing B-CLL tumor target cells, wells containing 1 nM of the T cell bispecific antibodies with effector cells but without B-CLL tumor target cells are also included. After incubation for five days at 37°C, 5% CO₂ cells are pelleted down by centrifugation (5 min, 350 x g) and washed twice with 150 µl/well of FACS Stain Buffer (BD Biosciences). Surface staining of the effector cells with selected fluorochrome-conjugated antibodies against human CD4, CD8 or CD25 (BD) is performed at 4°C for 30 min, protected from light, in FACS Stain Buffer according to the manufacturer's protocol. Cells are washed twice with 150 µl/well FACS Stain Buffer, resuspended in 200 µl/well FACS Stain Buffer, and acquired and analyzed using a LSR II flow cytometer complemented with FACSDiva® software (BD). The percentage of non-proliferating cells is determined by gating on the far right undiluted CFSE peak in the group which the wells contain ROR1-expressing B-CLL cells and CFSE-stained T cells but without the T cell bispecific antibodies, and compared that to other experimental groups (wells). The percentage of proliferating cells is measured by gating all the diluted CFSE peaks excluding the far right peak (if observable). The proliferation level of CD4⁺ and CD8⁺ T cells is determined by gating on that population first then to further look at the CFSE dilution peaks.

### Example 9 - Cytokine production from activated T cells upon engagement of anti-ROR1/anti-CD3 T cell bispecific antibodies

### Example 9A. Interferon-γ production

Anti-ROR1/anti-CD3 T cell bispecific antibodies generated in Example 3 are also analyzed for their potential to induce interferon-γ (IFN-γ) production by the T cells in the presence or absence of human ROR1-expressing B-CLL cells. Briefly, ROR1-expressing EHEB B-CLL cells are harvested with Cell Dissociation buffer, counted and looked for viability using Trypan Blue. Approximately 20,000 viable cells per well are plated in a round-bottom 96-well-plate and the respective antibody dilution is added to obtain final concentration(s) in the range of 0.1 pM to 200 nM. Anti-human ROR1 and anti-CD3 IgGs adjusted to the same molarity are used as controls. Human total T effector cells are added to obtain a final E:T ratio of 5:1. After 20 h incubation at 37°C, 5% CO₂, human IFN-γ levels in the supernatant are measured by ELISA, according to the manufacturer's instructions (human IFN-γ ELISA Kit II, BD Biosciences). The levels of IFN-γ produced by T cells in the presence of anti-ROR1/anti-CD3 T cell bispecific antibody and ROR1-expressing B-CLL cells is measured and plotted in histograms and compared to that produced by T cells in the presence of anti-ROR1/anti-CD3 T cell bispecific antibody and but without ROR1-expressing B-CLL cells.

### Example 9B. Cytokine release assay (CBA analysis)

Anti-ROR1/anti-CD3 T cell bispecific antibodies generated in Example 3 are also analyzed for their potential to induce T-cell mediated cytokine production in the presence or absence of human ROR1-expressing B-CLL cells. PBMC are isolated from fresh blood using density gradient centrifugation using Cell Preparation Tubes with Sodium citrate (Vacutainer CPT tubes, BD Biosciences) and a final cell concentration of 0.3 million cells/well are plated into a round-bottom 96-well plate. ROR1-expressing EHEB B-CLL cells are then added to obtain a final E:T-ratio of 10:1, as well as T cell bispecific constructs and IgG controls are added to obtain final concentration(s) in the range of 0.1 pM to 200 nM, for a 24 h incubation at 37°C, 5% CO₂. The next day, the cells are centrifuged for 5 min at 350 x g and the supernatant is transferred into a new deep-well 96-well-plate for the further analysis. The CBA analysis is performed according to manufacturer's instructions for LSR II flow cytometer, using the Human Th1/Th2 Cytokine Kit II (BD Biosciences) including human IL-2, human IL-4, human IL-6, human IL-10, human TNF-α, and human IFN-γ. The levels of cytokines produced by T cells in the presence of anti-ROR1/anti-CD3 T cell bispecific antibody and ROR1-expressing B-CLL cells is measured and plotted in histograms and compared to that produced by T cells in the presence of anti-ROR1/anti-CD3 T cell bispecific antibody and but without ROR1-expressing B-CLL cells.

### Example 10 - Redirected T cell cytotoxicity of B-CLL Cells upon cross-linking of anti-ROR1/anti-CD3 T cell bispecific antibodies to CD3 on T cells and ROR1 on B-CLL cells (LDH release assay)

Anti-ROR1/anti-CD3 T cell bispecific antibodies generated in Example 3 are also analyzed for their potential to induce T cell-mediated apoptosis in ROR1-expressing B-CLL cells upon crosslinking of the construct via binding of the antigen binding moieties to ROR1 on cells. Briefly, human ROR1-expressing EHEB B-CLL target cells are harvested with Cell Dissociation Buffer, washed and resuspended in RPMI supplemented with 10% fetal bovine serum (Invitrogen). Approximately, 30,000 cells per well are plated in a round-bottom 96-well plate and the respective dilution of the construct is added for a desired final concentration (in triplicates); final concentrations ranging from 0.1 pM to 200 nM. For an appropriate comparison, all T cell bispecific constructs and controls are adjusted to the same molarity. Human total T cells (effector) are added into the wells to obtain a final E:T ratio of 5:1. When human PBMC are used as effector cells, a final E:T ratio of 10:1 is used. PHA-L (Sigma) is used as positive control for human T cell activation at a concentration of 1 µg/ml. Negative control groups are represented by effector or target cells only. For normalization, maximal lysis of the EHEB B-CLL target cells (= 100%) is determined by incubation of the target cells with a final concentration of 1% Triton X-100, inducing cell death. Minimal lysis (= 0%) is represented by target cells co-incubated with effector cells only, i.e. without any T cell bispecific antibody. After 20 h incubation at 37°C, 5% CO₂, LDH release from the apoptotic/necrotic B-CLL target cells into the supernatant is then measured with the LDH detection kit (Roche Applied Science), following the manufacturer's instructions. The percentage of LDH release is plotted against the concentrations of anti-ROR1/anti-CD3 T cell bispecific antibodies in concentration-response curves. The IC₅₀ values are measured using Prism software (GraphPad) and determined as the T cell bispecific antibody concentration that results in 50% of LDH release.

### Example 11 - Redirected T cell cytotoxicity of multiple myeloma cells upon cross-linking of anti-ROR1/anti-CD3 T cell bispecific antibodies to CD3 on T cells and ROR1 on multiple myeloma cells (LDH release assay)

Anti-ROR1/anti-CD3 T cell bispecific antibodies generated in Example 3 are also analyzed for their potential to induce T cell-mediated apoptosis in ROR1-expressing multiple myeloma cells upon crosslinking of the construct via binding of the antigen binding moieties to ROR1 on cells. Briefly, human ROR1-expressing RPMI-8226 multiple myeloma target cells (available from American Type Culture Collection; ATCC CCL-155) are harvested with Cell Dissociation Buffer, washed and resuspended in RPMI supplemented with 10% fetal bovine serum (Invitrogen). Approximately, 30,000 cells per well are plated in a round-bottom 96-well plate and the respective dilution of the construct is added for a desired final concentration (in triplicates); final concentrations ranging from 0.1 pM to 200 nM. For an appropriate comparison, all T cell bispecific constructs and controls are adjusted to the same molarity. Human total T cells (effector) are added into the wells to obtain a final E:T ratio of 5:1. When human PBMC are used as effector cells, a final E:T ratio of 10:1 1 is used. PHA-L (Sigma) is used as positive control for human T cell activation at a concentration of 1 µg/ml. Negative control groups are represented by effector or target cells only. For normalization, maximal lysis of the RPMI-8226 multiple myeloma target cells (= 100%) is determined by incubation of the target cells with a final concentration of 1% Triton X-100, inducing cell death. Minimal lysis (= 0%) is represented by target cells co-incubated with effector cells only, i.e. without any T cell bispecific antibody. After 20 h incubation at 37°C, 5% CO₂, LDH release from the apoptotic/necrotic B-CLL target cells into the supernatant is then measured with the LDH detection kit (Roche Applied Science), following the manufacturer's instructions. The percentage of LDH release is plotted against the concentrations of anti-ROR1/anti-CD3 T cell bispecific antibodies in concentration-response curves. The IC₅₀ values are measured using Prism software (GraphPad) and determined as the T cell bispecific antibody concentration that results in 50% of LDH release.

### Example 12 - Redirected T cell cytotoxicity of cell lines from solid tumors upon cross-linking of anti-ROR1/anti-CD3 T cell bispecific antibodies to CD3 on T cells and ROR1 on solid tumor cells (LDH release assay)

Anti-ROR1/anti-CD3 T cell bispecific antibodies generated in Example 3 are also analyzed for their potential to induce T cell-mediated apoptosis in ROR1-expressing malignant cells from solid tumors (i.e. target cells) upon crosslinking of the construct via binding of the antigen binding moieties to ROR1 on cells. Briefly, human ROR1-expressing lung cancer cell line A549 (available from American Type Culture Collection; ATCC CCL-185) or breast cancer cell line MDA-MB-468 (available from American Type Culture Collection; ATCC HTB-132) are harvested with Cell Dissociation Buffer, washed and resuspended in RPMI supplemented with 10% fetal bovine serum (Invitrogen). Approximately, 30,000 cells per well are plated in a round-bottom 96-well plate and the respective dilution of the construct is added for a desired final concentration (in triplicates); final concentrations ranging from 0.1 pM to 200 nM. For an appropriate comparison, all T cell bispecific constructs and controls are adjusted to the same molarity. Human total T cells (effector) are added into the wells to obtain a final E:T ratio of 5:1. When human PBMC are used as effector cells, a final E:T ratio of 10:1 is used. PHA-L (Sigma) is used as positive control for human T cell activation at a concentration of 1 µg/ml. Negative control groups are represented by effector or target cells only. For normalization, maximal lysis of the lung cancer cell line A549 or breast cancer cell line MDA-MB-468 target cells (= 100%) is determined by incubation of the target cells with a final concentration of 1% Triton X-100, inducing cell death. Minimal lysis (= 0%) is represented by target cells co-incubated with effector cells only, i.e. without any T cell bispecific antibody. After 20 h incubation at 37°C, 5% CO₂, LDH release from the apoptotic/necrotic B-CLL target cells into the supernatant is then measured with the LDH detection kit (Roche Applied Science), following the manufacturer's instructions. The percentage of LDH release is plotted against the concentrations of anti-ROR1/anti-CD3 T cell bispecific antibodies in concentration-response curves. The IC₅₀ values are measured using Prism software (GraphPad) and determined as the T cell bispecific antibody concentration that results in 50% of LDH release.

### Example 13 - Evaluation of therapeutic efficacy of anti-ROR1/anti-CD3 T cell bispecific antibody in CLL xenograft SCID beige mouse model

Adult female CB17-severe combined immunodeficient (SCID) beige mice (Charles River Laboratories), are maintained under specific pathogen-free conditions according to guidelines. Continuous health monitoring is carried out on a regular basis. Mice are monitored daily for clinical symptoms and detection of adverse effects. Throughout the experimental period, the body weight of animals is recorded twice weekly and tumor volume is measured by caliper after staging. CB17-SCID mice are inoculated subcutaneously into their lower dorsum with EHEB B-CLL cells in 100 µL PBS and 100 µL Matrigel basement membrane matrix (Becton Dickinson). When tumors are palpable and reach ∼300 mm³, mice are randomly assigned into cohorts of 10 mice each and treated with ROR1-TCB 10-50 µg/mouse/ twice a week administered intravenously via the tail vein or an equal amount of control IgGs. Animals are sacrificed, according to institutional guidelines, when the diameter of their tumors reaches 3 cm or when significant toxicity is observed. Animal body weight and any sign of morbidity are monitored.

### Example 14 - Evaluation of therapeutic efficacy of anti-ROR1/anti-CD3 T cell bispecific antibody in the Vk*MYC multiple myeloma mouse model

Murine cross-reactive anti-ROR1/anti-CD3 T cell bispecific antibodies are tested for their potential to prevent multiple myeloma in Vk*MYC multiple myeloma prone mice as described in Chesi, 2012 (Chesi et al. 2012; Blood 120: 376-385). Multiple myeloma is a hematological malignancy involving an uncontrolled expansion of plasma cells in the bone marrow. Since ROR1 is strongly expressed on malignant plasma cells, we hypothesize that an anti-ROR1/anti-CD3 T cell bispecific will be efficacious for the treatment of multiple myeloma. The Vk*MYC multiple myeloma mouse model is highly representative of human myeloma and predictive of drug response used in the clinic; representing an excellent tool for testing the preclinical proof-of-concept of anti-ROR1/anti-CD3 T cell bispecific antibodies. Briefly, Vk*MYC mice obtained from an academic collaboration at Mayo Clinic Arizona are crossed with human CD3ε transgenic (huCD3ε Tg) mice. T cells from huCD3ε Tg x Vk*MYC mice express both human CD3ε and mouse CD3ε on the cell surface and the mice are therefore responsive to anti-ROR1/anti-CD3 T cell bispecifics. Vk*MYC mice uniformly develop a monoclonal gammopathy initiated at around 30 weeks of age that progresses slowly over time associated with clinical signs representative of human myeloma such as anemia, osteoporosis and renal disease. Mice are periodically bled by tail grazing and blood is collected into Microtainer tubes (BD Biosciences), let coagulate at room temperature then spun for 10 min at 2,300g. Sera are diluted 1:2 in normal saline buffer and analyzed on a QuickGel Chamber apparatus using pre-casted QuickGels (Helena Laboratories) according to manufacturer's instruction. Gamma/albumin ratio and serum fractions are measured by densitometric analysis.

For therapeutic studies, Vk*MYC mice are enrolled and randomized into different treatment groups (n=5-8/group): for example, 1) control IgGs; 2) anti-ROR1/anti-CD3 T cell bispecific antibodies; 500 µg/kg/week or 10 µg/mouse/week administered intravenously via the tail vein; 3) bortezomib 1 mg/kg/ i.p. on days 1,4,8,11 used as standard of care. Preferably, the dose(s) of anti-ROR1/anti-CD3 T cell bispecific antibodies could be multiple and range from 200 to 1000 µg/kg/week. In each group, at least three aged (>1 year old) Vk*MYC mice with gamma/albumin ratio between 0.3-2.0, corresponding to a predominant M-spike between approximately 10-70 g/l as measured by densitometry. Serum protein electrophoresis (SPEP) is performed on day 0 and day 14 post treatment to measure treatment-mediated reduction in the M-spike as a marker of tumor response, as done in the clinic. In some therapeutic studies, transplanted Vk*MYC mice with an M-spike approximately 10-70 g/l and a bone marrow plasmacytosis of greater than 5% are enrolled and assigned to different treatment groups. The efficacy of anti-ROR1/anti-CD3 T cell bispecific antibodies to reduce M-spike is evaluated.

## Claims

1. A bispecific antibody specifically binding to the two targets human CD3ε (further named also as "CD3") and the extracellular domain of human ROR1 (further named also as "ROR1"), **characterized in that** the bispecific antibody does not internalize in a cell based assay at 37°C during 2 hrs., using ROR1-positive primary B-CLL cells and used at an antibody concentration of 1 nM, the Δ mean fluorescence intensity (ΔMFI), MFI as measured by flow cytometry, reflecting the internalization (%) of the said antibody to ROR1-positive cells is between 120% to 0%of the ΔMFI (internalization, %) of an anti-ROR1 bivalent antibody of human IgG1 kappa (κ) type comprising as light chain variable domain (VL) the sequence of SEQ ID NO:2 and as variable heavy chain domain (VH) the sequence of SEQ ID NO:6, in the same concentration and experimental conditions.

2. A bispecific antibody specifically binding to the two targets human CD3ε (further named also as "CD3") and the extracellular domain of human ROR1 (further named also as "ROR1"), **characterized in that** the bispecific antibody does not internalize in a cell based assay at 37°C during 2 hrs., using ROR1-positive EHEB B-CLL cell line DSMZ ACC-67 and used at an antibody concentration of 1 nM, the Δ mean fluorescence intensity (ΔMFI), MFI as measured by flow cytometry, reflecting the internalization (%) of the said antibody to ROR1-positive cells is between 120% to 0%of the ΔMFI (internalization, %) of an anti-ROR1 bivalent antibody of human IgG1 kappa (κ) type comprising as light chain variable domain (VL) the sequence of SEQ ID NO:2 and as variable heavy chain domain (VH) the sequence of SEQ ID NO:6, in the same concentration and experimental conditions.

3. A bispecific bivalent antibody according to claim 1or 2, **characterized in** comprising a monovalent anti-ROR1 antibody specifically binding to ROR1, and a monovalent antibody specifically binding to CD3.

4. A bispecific trivalent antibody according to claim 1or 2, **characterized in** comprising a bispecific anti-ROR1 antibody specifically binding to ROR1, and a monovalent Fab fragment or a single chain Fv fragment of an antibody specifically binding to CD3.

5. A bispecific antibody according to any one of claims 1 to 4, **characterized in** being devoid of a Fc domain.

6. A bispecific antibody to any one of claims 1 to 5, **characterized in** comprising
a) the light chain and heavy chain of an antibody specifically binding to one of said targets; and
b) the light chain and heavy chain of an antibody specifically binding to the other one of said targets, wherein the variable domains VL and VH or the constant domains CL and CH1 are replaced by each other.

7. A bispecific antibody according to any one of claims 4 to 6, **characterized in**
a) consisting of a F(ab)₂ fragment of an anti-ROR1 antibody specifically binding to ROR1, wherein in one arm the variable domains VL and VH or the constant domains CL and CH1 are replaced by each other, with or without Fc part, and
b) consisting of a Fab fragment or a single chain Fv fragment of an antibody specifically binding to CD3 bound to the N-terminus of one VL or VH domain of said antibody F(ab)₂ fragment specifically binding to ROR1.

8. A bispecific antibody according to any one of claims 1 to 6, **characterized in that** the antibody portion specifically binding to human CD3ε is **characterized in** comprising a variable heavy chain domain VH comprising the CDRs of SEQ ID NO: 12, 13 and 14 as respectively heavy chain CDR1, CDR2 and CDR3 and a variable domain VL comprising the CDRs of SEQ ID NO: 15, 16 and 17 as respectively light chain CDR1, CDR2 and CDR3.

9. A bispecific antibody according to any one of claims 1 to 7, **characterized in that** the antibody portion specifically binding to human ROR1 is **characterized in** comprising a variable heavy chain domain VH comprising the CDRs of SEQ ID NO: 7, 8 and 9 as respectively heavy chain CDR1, CDR2 and CDR3 and a variable domain VL comprising the CDRs of SEQ ID NO: 3, 4 and 5 as respectively light chain CDR1, CDR2 and CDR3

10. A method for the preparation of a bispecific antibody according to any one of claims 1 to 9 comprising the steps of transforming a host cell with one or more vectors comprising nucleic acid molecules encoding the respective antibodies and/or fragments and culturing the host cell under conditions that allow synthesis of said antibody molecule; and recovering said antibody molecule from said culture.

11. A host cell comprising vectors comprising nucleic acid molecules encoding the light chain and heavy chains of an antibody according to any one of claims 1 to 9.

12. A pharmaceutical composition comprising an antibody according to any one of claims 1 to 9 and a pharmaceutically acceptable excipient.

13. An antibody according to any one of claims 1 to 9 or the pharmaceutical composition of claim 10 for use as a medicament.

14. An antibody according to any one of claims 1 to 9 or the pharmaceutical composition of claim 12 for use as a medicament in the treatment of ROR1-positive hematological malignancies comprising chronic lymphocytic leukemia (CLL), hairy cell leukemia (HCL), acute lymphoblastic leukemia (ALL), acute myeloid leukemia (AML), chronic myeloid leukemia (CML), mantle cell lymphoma (MCL), marginal zone lymphoma (MZL), diffuse large B cell lymphoma (DLBCL), multiple myeloma (MM), follicular lymphoma (FL), and for the treatment of ROR1-positive solid tumors such as breast cancer and lung cancer.

15. An antibody according to any one of claims 1 to 9 or the pharmaceutical composition of claim 12 for use as a medicament in the treatment of treatment of chronic lymphocytic leukemia (CLL) of B-cell lineage (B-CLL) and for use as a medicament in the treatment of plasma cell disorders like Multiple Myeloma MM or other B-cell disorders expressing ROR1.


SEQUENCE LISTING

<110>  VU, Minh Diem
       Strein, Klaus

<120>  Bispecific antibodies against CD3 and ROR1

<130>  ROR-EP

<160>  20

<170>  PatentIn version 3.5

<210>  1
<211>  937
<212>  PRT
<213>  human

<400>  1

Met His Arg Pro Arg Arg Arg Gly Thr Arg Pro Pro Leu Leu Ala Leu
1               5                   10                  15

Leu Ala Ala Leu Leu Leu Ala Ala Arg Gly Ala Ala Ala Gln Glu Thr
            20                  25                  30

Glu Leu Ser Val Ser Ala Glu Leu Val Pro Thr Ser Ser Trp Asn Ile
        35                  40                  45

Ser Ser Glu Leu Asn Lys Asp Ser Tyr Leu Thr Leu Asp Glu Pro Met
    50                  55                  60

Asn Asn Ile Thr Thr Ser Leu Gly Gln Thr Ala Glu Leu His Cys Lys
65                  70                  75                  80

Val Ser Gly Asn Pro Pro Pro Thr Ile Arg Trp Phe Lys Asn Asp Ala
                85                  90                  95

Pro Val Val Gln Glu Pro Arg Arg Leu Ser Phe Arg Ser Thr Ile Tyr
            100                 105                 110

Gly Ser Arg Leu Arg Ile Arg Asn Leu Asp Thr Thr Asp Thr Gly Tyr
        115                 120                 125

Phe Gln Cys Val Ala Thr Asn Gly Lys Glu Val Val Ser Ser Thr Gly
    130                 135                 140

Val Leu Phe Val Lys Phe Gly Pro Pro Pro Thr Ala Ser Pro Gly Tyr
145                 150                 155                 160

Ser Asp Glu Tyr Glu Glu Asp Gly Phe Cys Gln Pro Tyr Arg Gly Ile
                165                 170                 175

Ala Cys Ala Arg Phe Ile Gly Asn Arg Thr Val Tyr Met Glu Ser Leu
            180                 185                 190

His Met Gln Gly Glu Ile Glu Asn Gln Ile Thr Ala Ala Phe Thr Met
        195                 200                 205

Ile Gly Thr Ser Ser His Leu Ser Asp Lys Cys Ser Gln Phe Ala Ile
    210                 215                 220

Pro Ser Leu Cys His Tyr Ala Phe Pro Tyr Cys Asp Glu Thr Ser Ser
225                 230                 235                 240

Val Pro Lys Pro Arg Asp Leu Cys Arg Asp Glu Cys Glu Ile Leu Glu
                245                 250                 255

Asn Val Leu Cys Gln Thr Glu Tyr Ile Phe Ala Arg Ser Asn Pro Met
            260                 265                 270

Ile Leu Met Arg Leu Lys Leu Pro Asn Cys Glu Asp Leu Pro Gln Pro
        275                 280                 285

Glu Ser Pro Glu Ala Ala Asn Cys Ile Arg Ile Gly Ile Pro Met Ala
    290                 295                 300

Asp Pro Ile Asn Lys Asn His Lys Cys Tyr Asn Ser Thr Gly Val Asp
305                 310                 315                 320

Tyr Arg Gly Thr Val Ser Val Thr Lys Ser Gly Arg Gln Cys Gln Pro
                325                 330                 335

Trp Asn Ser Gln Tyr Pro His Thr His Thr Phe Thr Ala Leu Arg Phe
            340                 345                 350

Pro Glu Leu Asn Gly Gly His Ser Tyr Cys Arg Asn Pro Gly Asn Gln
        355                 360                 365

Lys Glu Ala Pro Trp Cys Phe Thr Leu Asp Glu Asn Phe Lys Ser Asp
    370                 375                 380

Leu Cys Asp Ile Pro Ala Cys Asp Ser Lys Asp Ser Lys Glu Lys Asn
385                 390                 395                 400

Lys Met Glu Ile Leu Tyr Ile Leu Val Pro Ser Val Ala Ile Pro Leu
                405                 410                 415

Ala Ile Ala Leu Leu Phe Phe Phe Ile Cys Val Cys Arg Asn Asn Gln
            420                 425                 430

Lys Ser Ser Ser Ala Pro Val Gln Arg Gln Pro Lys His Val Arg Gly
        435                 440                 445

Gln Asn Val Glu Met Ser Met Leu Asn Ala Tyr Lys Pro Lys Ser Lys
    450                 455                 460

Ala Lys Glu Leu Pro Leu Ser Ala Val Arg Phe Met Glu Glu Leu Gly
465                 470                 475                 480

Glu Cys Ala Phe Gly Lys Ile Tyr Lys Gly His Leu Tyr Leu Pro Gly
                485                 490                 495

Met Asp His Ala Gln Leu Val Ala Ile Lys Thr Leu Lys Asp Tyr Asn
            500                 505                 510

Asn Pro Gln Gln Trp Thr Glu Phe Gln Gln Glu Ala Ser Leu Met Ala
        515                 520                 525

Glu Leu His His Pro Asn Ile Val Cys Leu Leu Gly Ala Val Thr Gln
    530                 535                 540

Glu Gln Pro Val Cys Met Leu Phe Glu Tyr Ile Asn Gln Gly Asp Leu
545                 550                 555                 560

His Glu Phe Leu Ile Met Arg Ser Pro His Ser Asp Val Gly Cys Ser
                565                 570                 575

Ser Asp Glu Asp Gly Thr Val Lys Ser Ser Leu Asp His Gly Asp Phe
            580                 585                 590

Leu His Ile Ala Ile Gln Ile Ala Ala Gly Met Glu Tyr Leu Ser Ser
        595                 600                 605

His Phe Phe Val His Lys Asp Leu Ala Ala Arg Asn Ile Leu Ile Gly
    610                 615                 620

Glu Gln Leu His Val Lys Ile Ser Asp Leu Gly Leu Ser Arg Glu Ile
625                 630                 635                 640

Tyr Ser Ala Asp Tyr Tyr Arg Val Gln Ser Lys Ser Leu Leu Pro Ile
                645                 650                 655

Arg Trp Met Pro Pro Glu Ala Ile Met Tyr Gly Lys Phe Ser Ser Asp
            660                 665                 670

Ser Asp Ile Trp Ser Phe Gly Val Val Leu Trp Glu Ile Phe Ser Phe
        675                 680                 685

Gly Leu Gln Pro Tyr Tyr Gly Phe Ser Asn Gln Glu Val Ile Glu Met
    690                 695                 700

Val Arg Lys Arg Gln Leu Leu Pro Cys Ser Glu Asp Cys Pro Pro Arg
705                 710                 715                 720

Met Tyr Ser Leu Met Thr Glu Cys Trp Asn Glu Ile Pro Ser Arg Arg
                725                 730                 735

Pro Arg Phe Lys Asp Ile His Val Arg Leu Arg Ser Trp Glu Gly Leu
            740                 745                 750

Ser Ser His Thr Ser Ser Thr Thr Pro Ser Gly Gly Asn Ala Thr Thr
        755                 760                 765

Gln Thr Thr Ser Leu Ser Ala Ser Pro Val Ser Asn Leu Ser Asn Pro
    770                 775                 780

Arg Tyr Pro Asn Tyr Met Phe Pro Ser Gln Gly Ile Thr Pro Gln Gly
785                 790                 795                 800

Gln Ile Ala Gly Phe Ile Gly Pro Pro Ile Pro Gln Asn Gln Arg Phe
                805                 810                 815

Ile Pro Ile Asn Gly Tyr Pro Ile Pro Pro Gly Tyr Ala Ala Phe Pro
            820                 825                 830

Ala Ala His Tyr Gln Pro Thr Gly Pro Pro Arg Val Ile Gln His Cys
        835                 840                 845

Pro Pro Pro Lys Ser Arg Ser Pro Ser Ser Ala Ser Gly Ser Thr Ser
    850                 855                 860

Thr Gly His Val Thr Ser Leu Pro Ser Ser Gly Ser Asn Gln Glu Ala
865                 870                 875                 880

Asn Ile Pro Leu Leu Pro His Met Ser Ile Pro Asn His Pro Gly Gly
                885                 890                 895

Met Gly Ile Thr Val Phe Gly Asn Lys Ser Gln Lys Pro Tyr Lys Ile
            900                 905                 910

Asp Ser Lys Gln Ala Ser Leu Leu Gly Asp Ala Asn Ile His Gly His
        915                 920                 925

Thr Glu Ser Met Ile Ser Ala Glu Leu
    930                 935

<210>  2
<211>  112
<212>  PRT
<213>  murine

<400>  2

Glu Leu Val Leu Thr Gln Ser Pro Ser Val Ser Ala Ala Leu Gly Ser
1               5                   10                  15

Pro Ala Lys Ile Thr Cys Thr Leu Ser Ser Ala His Lys Thr Asp Thr
            20                  25                  30

Ile Asp Trp Tyr Gln Gln Leu Gln Gly Glu Ala Pro Arg Tyr Leu Met

35                  40                  45

Gln Val Gln Ser Asp Gly Ser Tyr Thr Lys Arg Pro Gly Val Pro Asp
    50                  55                  60

Arg Phe Ser Gly Ser Ser Ser Gly Ala Asp Arg Tyr Leu Ile Ile Pro
65                  70                  75                  80

Ser Val Gln Ala Asp Asp Glu Ala Asp Tyr Tyr Cys Gly Ala Asp Tyr
                85                  90                  95

Ile Gly Gly Tyr Val Phe Gly Gly Gly Thr Gln Leu Thr Val Thr Gly
            100                 105                 110

<210>  3
<211>  12
<212>  PRT
<213>  murine

<400>  3

Thr Leu Ser Ser Ala His Lys Thr Asp Thr Ile Asp
1               5                   10

<210>  4
<211>  7
<212>  PRT
<213>  murine

<400>  4

Gly Ser Tyr Thr Lys Arg Pro
1               5

<210>  5
<211>  9
<212>  PRT
<213>  murine

<400>  5

Gly Ala Asp Tyr Ile Gly Gly Tyr Val
1               5

<210>  6
<211>  121
<212>  PRT
<213>  murine

<400>  6

Gln Glu Gln Leu Val Glu Ser Gly Gly Arg Leu Val Thr Pro Gly Gly
1               5                   10                  15

Ser Leu Thr Leu Ser Cys Lys Ala Ser Gly Phe Asp Phe Ser Ala Tyr
            20                  25                  30

Tyr Met Ser Trp Val Arg Gln Ala Pro Gly Lys Gly Leu Glu Trp Ile

35                  40                  45

Ala Thr Ile Tyr Pro Ser Ser Gly Lys Thr Tyr Tyr Ala Thr Trp Val
    50                  55                  60

Asn Gly Arg Phe Thr Ile Ser Ser Asp Asn Ala Gln Asn Thr Val Asp
65                  70                  75                  80

Leu Gln Met Asn Ser Leu Thr Ala Ala Asp Arg Ala Thr Tyr Phe Cys
                85                  90                  95

Ala Arg Asp Ser Tyr Ala Asp Asp Gly Ala Leu Phe Asn Ile Trp Gly
            100                 105                 110

Pro Gly Thr Leu Val Thr Ile Ser Ser
        115                 120

<210>  7
<211>  5
<212>  PRT
<213>  murine

<400>  7

Ala Tyr Tyr Met Ser
1               5

<210>  8
<211>  17
<212>  PRT
<213>  murine

<400>  8

Thr Ile Tyr Pro Ser Ser Gly Lys Thr Tyr Tyr Ala Thr Trp Val Asn
1               5                   10                  15

Gly

<210>  9
<211>  12
<212>  PRT
<213>  murine

<400>  9

Asp Ser Tyr Ala Asp Asp Gly Ala Leu Phe Asn Ile
1               5                   10

<210>  10
<211>  125
<212>  PRT
<213>  murine

<400>  10

Glu Val Gln Leu Val Glu Ser Gly Gly Gly Leu Val Gln Pro Gly Gly

1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210>  11
<211>  109
<212>  PRT
<213>  murine

<400>  11

Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Tyr Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Arg Trp Val Phe Gly Gly Gly Thr Lys Leu Thr Val Leu
            100                 105

<210>  12
<211>  10

<212>  PRT
<213>  murine

<400>  12

Gly Phe Thr Phe Asn Lys Tyr Ala Met Asn
1               5                   10


<210>  13
<211>  19
<212>  PRT
<213>  murine

<400>  13

Arg Ile Arg Ser Lys Tyr Asn Asn Tyr Ala Thr Tyr Tyr Ala Asp Ser
1               5                   10                  15


Val Lys Asp



<210>  14
<211>  14
<212>  PRT
<213>  murine

<400>  14

His Gly Asn Phe Gly Asn Ser Tyr Ile Ser Tyr Trp Ala Tyr
1               5                   10


<210>  15
<211>  14
<212>  PRT
<213>  murine

<400>  15

Gly Ser Ser Thr Gly Ala Val Thr Ser Gly Tyr Tyr Pro Asn
1               5                   10


<210>  16
<211>  7
<212>  PRT
<213>  murine

<400>  16

Gly Thr Lys Phe Leu Ala Pro
1               5


<210>  17
<211>  9
<212>  PRT
<213>  murine

<400>  17

Ala Leu Trp Tyr Ser Asn Arg Trp Val
1               5

<210>  18
<211>  15
<212>  PRT
<213>  human

<400>  18

Trp Asn Ile Ser Ser Glu Leu Asn Lys Asp Ser Tyr Leu Thr Leu
1               5                   10                  15


<210>  19
<211>  10
<212>  PRT
<213>  artificial

<220>
<223>  artificial linker sequence

<400>  19

Gly Gly Gly Gly Ser Gly Gly Gly Gly Ser
1               5                   10


<210>  20
<211>  14
<212>  PRT
<213>  human

<400>  20

Lys Ser Gln Lys Pro Tyr Lys Ile Asp Ser Lys Gln Ala Ser
1               5                   10

SEQUENCE LISTING

<110> VU, Minh Diem
      Strein, Klaus

<120> Bispecific antibodies against CD3 and ROR1

<130> ROR-EP

<160> 20

<170> PatentIn version 3.5

<210> 1
<211> 937
<212> PRT
<213> human

<400> 1

Met His Arg Pro Arg Arg Arg Gly Thr Arg Pro Pro Leu Leu Ala Leu
1               5                   10                  15

Leu Ala Ala Leu Leu Leu Ala Ala Arg Gly Ala Ala Ala Gln Glu Thr
            20                  25                  30

Glu Leu Ser Val Ser Ala Glu Leu Val Pro Thr Ser Ser Trp Asn Ile
        35                  40                  45

Ser Ser Glu Leu Asn Lys Asp Ser Tyr Leu Thr Leu Asp Glu Pro Met
    50                  55                  60

Asn Asn Ile Thr Thr Ser Leu Gly Gln Thr Ala Glu Leu His Cys Lys
65                  70                  75                  80

Val Ser Gly Asn Pro Pro Pro Thr Ile Arg Trp Phe Lys Asn Asp Ala
                85                  90                  95

Pro Val Val Gln Glu Pro Arg Arg Leu Ser Phe Arg Ser Thr Ile Tyr
            100                 105                 110

Gly Ser Arg Leu Arg Ile Arg Asn Leu Asp Thr Thr Asp Thr Gly Tyr
        115                 120                 125

Phe Gln Cys Val Ala Thr Asn Gly Lys Glu Val Val Ser Ser Thr Gly
    130                 135                 140

Val Leu Phe Val Lys Phe Gly Pro Pro Pro Thr Ala Ser Pro Gly Tyr
145                 150                 155                 160

Ser Asp Glu Tyr Glu Glu Asp Gly Phe Cys Gln Pro Tyr Arg Gly Ile
                165                 170                 175

Ala Cys Ala Arg Phe Ile Gly Asn Arg Thr Val Tyr Met Glu Ser Leu
            180                 185                 190

His Met Gln Gly Glu Ile Glu Asn Gln Ile Thr Ala Ala Phe Thr Met
        195                 200                 205

Ile Gly Thr Ser Ser His Leu Ser Asp Lys Cys Ser Gln Phe Ala Ile
    210                 215                 220

Pro Ser Leu Cys His Tyr Ala Phe Pro Tyr Cys Asp Glu Thr Ser Ser
225                 230                 235                 240

Val Pro Lys Pro Arg Asp Leu Cys Arg Asp Glu Cys Glu Ile Leu Glu
                245                 250                 255

Asn Val Leu Cys Gln Thr Glu Tyr Ile Phe Ala Arg Ser Asn Pro Met
            260                 265                 270

Ile Leu Met Arg Leu Lys Leu Pro Asn Cys Glu Asp Leu Pro Gln Pro
        275                 280                 285

Glu Ser Pro Glu Ala Ala Asn Cys Ile Arg Ile Gly Ile Pro Met Ala
    290                 295                 300

Asp Pro Ile Asn Lys Asn His Lys Cys Tyr Asn Ser Thr Gly Val Asp
305                 310                 315                 320

Tyr Arg Gly Thr Val Ser Val Thr Lys Ser Gly Arg Gln Cys Gln Pro
                325                 330                 335

Trp Asn Ser Gln Tyr Pro His Thr His Thr Phe Thr Ala Leu Arg Phe
            340                 345                 350

Pro Glu Leu Asn Gly Gly His Ser Tyr Cys Arg Asn Pro Gly Asn Gln
        355                 360                 365

Lys Glu Ala Pro Trp Cys Phe Thr Leu Asp Glu Asn Phe Lys Ser Asp
    370                 375                 380

Leu Cys Asp Ile Pro Ala Cys Asp Ser Lys Asp Ser Lys Glu Lys Asn
385                 390                 395                 400

Lys Met Glu Ile Leu Tyr Ile Leu Val Pro Ser Val Ala Ile Pro Leu
                405                 410                 415

Ala Ile Ala Leu Leu Phe Phe Phe Ile Cys Val Cys Arg Asn Asn Gln
            420                 425                 430

Lys Ser Ser Ser Ala Pro Val Gln Arg Gln Pro Lys His Val Arg Gly
        435                 440                 445

Gln Asn Val Glu Met Ser Met Leu Asn Ala Tyr Lys Pro Lys Ser Lys
    450                 455                 460

Ala Lys Glu Leu Pro Leu Ser Ala Val Arg Phe Met Glu Glu Leu Gly
465                 470                 475                 480

Glu Cys Ala Phe Gly Lys Ile Tyr Lys Gly His Leu Tyr Leu Pro Gly
                485                 490                 495

Met Asp His Ala Gln Leu Val Ala Ile Lys Thr Leu Lys Asp Tyr Asn
            500                 505                 510

Asn Pro Gln Gln Trp Thr Glu Phe Gln Gln Glu Ala Ser Leu Met Ala
        515                 520                 525

Glu Leu His His Pro Asn Ile Val Cys Leu Leu Gly Ala Val Thr Gln
    530                 535                 540

Glu Gln Pro Val Cys Met Leu Phe Glu Tyr Ile Asn Gln Gly Asp Leu
545                 550                 555                 560

His Glu Phe Leu Ile Met Arg Ser Pro His Ser Asp Val Gly Cys Ser
                565                 570                 575

Ser Asp Glu Asp Gly Thr Val Lys Ser Ser Leu Asp His Gly Asp Phe
            580                 585                 590

Leu His Ile Ala Ile Gln Ile Ala Ala Gly Met Glu Tyr Leu Ser Ser
        595                 600                 605

His Phe Phe Val His Lys Asp Leu Ala Ala Arg Asn Ile Leu Ile Gly
    610                 615                 620

Glu Gln Leu His Val Lys Ile Ser Asp Leu Gly Leu Ser Arg Glu Ile
625                 630                 635                 640

Tyr Ser Ala Asp Tyr Tyr Arg Val Gln Ser Lys Ser Leu Leu Pro Ile
                645                 650                 655

Arg Trp Met Pro Pro Glu Ala Ile Met Tyr Gly Lys Phe Ser Ser Asp
            660                 665                 670

Ser Asp Ile Trp Ser Phe Gly Val Val Leu Trp Glu Ile Phe Ser Phe

675                 680                 685

Gly Leu Gln Pro Tyr Tyr Gly Phe Ser Asn Gln Glu Val Ile Glu Met
    690                 695                 700

Val Arg Lys Arg Gln Leu Leu Pro Cys Ser Glu Asp Cys Pro Pro Arg
705                 710                 715                 720

Met Tyr Ser Leu Met Thr Glu Cys Trp Asn Glu Ile Pro Ser Arg Arg
                725                 730                 735

Pro Arg Phe Lys Asp Ile His Val Arg Leu Arg Ser Trp Glu Gly Leu
            740                 745                 750

Ser Ser His Thr Ser Ser Thr Thr Pro Ser Gly Gly Asn Ala Thr Thr
        755                 760                 765

Gln Thr Thr Ser Leu Ser Ala Ser Pro Val Ser Asn Leu Ser Asn Pro
    770                 775                 780

Arg Tyr Pro Asn Tyr Met Phe Pro Ser Gln Gly Ile Thr Pro Gln Gly
785                 790                 795                 800

Gln Ile Ala Gly Phe Ile Gly Pro Pro Ile Pro Gln Asn Gln Arg Phe
                805                 810                 815

Ile Pro Ile Asn Gly Tyr Pro Ile Pro Pro Gly Tyr Ala Ala Phe Pro
            820                 825                 830

Ala Ala His Tyr Gln Pro Thr Gly Pro Pro Arg Val Ile Gln His Cys
        835                 840                 845

Pro Pro Pro Lys Ser Arg Ser Pro Ser Ser Ala Ser Gly Ser Thr Ser
    850                 855                 860

Thr Gly His Val Thr Ser Leu Pro Ser Ser Gly Ser Asn Gln Glu Ala
865                 870                 875                 880

Asn Ile Pro Leu Leu Pro His Met Ser Ile Pro Asn His Pro Gly Gly
                885                 890                 895

Met Gly Ile Thr Val Phe Gly Asn Lys Ser Gln Lys Pro Tyr Lys Ile
            900                 905                 910

Asp Ser Lys Gln Ala Ser Leu Leu Gly Asp Ala Asn Ile His Gly His
        915                 920                 925

Thr Glu Ser Met Ile Ser Ala Glu Leu
    930                 935

<210>  2
<211>  112
<212>  PRT
<213>  murine

<400>  2

Glu Leu Val Leu Thr Gln Ser Pro Ser Val Ser Ala Ala Leu Gly Ser
1               5                   10                  15

Pro Ala Lys Ile Thr Cys Thr Leu Ser Ser Ala His Lys Thr Asp Thr
            20                  25                  30

Ile Asp Trp Tyr Gln Gln Leu Gln Gly Glu Ala Pro Arg Tyr Leu Met
        35                  40                  45

Gln Val Gln Ser Asp Gly Ser Tyr Thr Lys Arg Pro Gly Val Pro Asp
    50                  55                  60

Arg Phe Ser Gly Ser Ser Ser Gly Ala Asp Arg Tyr Leu Ile Ile Pro
65                  70                  75                  80

Ser Val Gln Ala Asp Asp Glu Ala Asp Tyr Tyr Cys Gly Ala Asp Tyr
                85                  90                  95

Ile Gly Gly Tyr Val Phe Gly Gly Gly Thr Gln Leu Thr Val Thr Gly
            100                 105                 110

<210>  3
<211>  12
<212>  PRT
<213>  murine

<400>  3

Thr Leu Ser Ser Ala His Lys Thr Asp Thr Ile Asp
1               5                   10

<210>  4
<211>  7
<212>  PRT
<213>  murine

<400>  4

Gly Ser Tyr Thr Lys Arg Pro
1               5

<210>  5
<211>  9

<212>  PRT
<213>  murine

<400>  5

Gly Ala Asp Tyr Ile Gly Gly Tyr Val
1               5


<210>  6
<211>  121
<212>  PRT
<213>  murine

<400>  6

Gln Glu Gln Leu Val Glu Ser Gly Gly Arg Leu Val Thr Pro Gly Gly
1               5                   10                  15


Ser Leu Thr Leu Ser Cys Lys Ala Ser Gly Phe Asp Phe Ser Ala Tyr
            20                  25                  30


Tyr Met Ser Trp Val Arg Gln Ala Pro Gly Lys Gly Leu Glu Trp Ile
        35                  40                  45


Ala Thr Ile Tyr Pro Ser Ser Gly Lys Thr Tyr Tyr Ala Thr Trp Val
    50                  55                  60


Asn Gly Arg Phe Thr Ile Ser Ser Asp Asn Ala Gln Asn Thr Val Asp
65                  70                  75                  80


Leu Gln Met Asn Ser Leu Thr Ala Ala Asp Arg Ala Thr Tyr Phe Cys
                85                  90                  95


Ala Arg Asp Ser Tyr Ala Asp Asp Gly Ala Leu Phe Asn Ile Trp Gly
            100                 105                 110


Pro Gly Thr Leu Val Thr Ile Ser Ser
        115                 120


<210>  7
<211>  5
<212>  PRT
<213>  murine

<400>  7

Ala Tyr Tyr Met Ser
1               5


<210>  8
<211>  17
<212>  PRT
<213>  murine

<400>  8

Thr Ile Tyr Pro Ser Ser Gly Lys Thr Tyr Tyr Ala Thr Trp Val Asn
1               5                   10                  15

Gly

<210>  9
<211>  12
<212>  PRT
<213>  murine

<400>  9

Asp Ser Tyr Ala Asp Asp Gly Ala Leu Phe Asn Ile
1               5                   10

<210>  10
<211>  125
<212>  PRT
<213>  murine

<400>  10

Glu Val Gln Leu Val Glu Ser Gly Gly Gly Leu Val Gln Pro Gly Gly
1               5                   10                  15

Ser Leu Lys Leu Ser Cys Ala Ala Ser Gly Phe Thr Phe Asn Lys Tyr
            20                  25                  30

Ala Met Asn Trp Val Arg Gln Ala Pro Gly Lys Gly Leu Glu Trp Val
        35                  40                  45

Ala Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Thr
65                  70                  75                  80

Ala Tyr Leu Gln Met Asn Asn Leu Lys Thr Glu Asp Thr Ala Val Tyr
                85                  90                  95

Tyr Cys Val Arg His Gly Asn Phe Gly Asn Ser Tyr Ile Ser Tyr Trp
            100                 105                 110

Ala Tyr Trp Gly Gln Gly Thr Leu Val Thr Val Ser Ser
        115                 120                 125

<210>  11
<211>  109

<212>  PRT
<213>  murine

<400>  11

Gln Thr Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Ser Gly
            20                  25                  30

Tyr Tyr Pro Asn Trp Val Gln Gln Lys Pro Gly Gln Ala Pro Arg Gly
        35                  40                  45

Leu Ile Gly Gly Thr Lys Phe Leu Ala Pro Gly Thr Pro Ala Arg Phe
    50                  55                  60

Ser Gly Ser Leu Leu Gly Gly Lys Ala Ala Leu Thr Leu Ser Gly Val
65                  70                  75                  80

Gln Pro Glu Asp Glu Ala Glu Tyr Tyr Cys Ala Leu Trp Tyr Ser Asn
                85                  90                  95

Arg Trp Val Phe Gly Gly Gly Thr Lys Leu Thr Val Leu
            100                 105

<210>  12
<211>  10
<212>  PRT
<213>  murine

<400>  12

Gly Phe Thr Phe Asn Lys Tyr Ala Met Asn
1               5                   10

<210>  13
<211>  19
<212>  PRT
<213>  murine

<400>  13

Arg Ile Arg Ser Lys Tyr Asn Asn Tyr Ala Thr Tyr Tyr Ala Asp Ser
1               5                   10                  15

Val Lys Asp

<210>  14
<211>  14
<212>  PRT
<213>  murine

<400>  14

His Gly Asn Phe Gly Asn Ser Tyr Ile Ser Tyr Trp Ala Tyr
1               5                   10


<210>  15
<211>  14
<212>  PRT
<213>  murine

<400>  15

Gly Ser Ser Thr Gly Ala Val Thr Ser Gly Tyr Tyr Pro Asn
1               5                   10


<210>  16
<211>  7
<212>  PRT
<213>  murine

<400>  16

Gly Thr Lys Phe Leu Ala Pro
1               5


<210>  17
<211>  9
<212>  PRT
<213>  murine

<400>  17

Ala Leu Trp Tyr Ser Asn Arg Trp Val
1               5


<210>  18
<211>  15
<212>  PRT
<213>  human

<400>  18

Trp Asn Ile Ser Ser Glu Leu Asn Lys Asp Ser Tyr Leu Thr Leu
1               5                   10                  15


<210>  19
<211>  10
<212>  PRT
<213>  artificial

<220>
<223>  artificial linker sequence

<400>  19

Gly Gly Gly Gly Ser Gly Gly Gly Gly Ser
1               5                   10

<210>  20
<211>  14
<212>  PRT
<213>  human

<400>  20

Lys Ser Gln Lys Pro Tyr Lys Ile Asp Ser Lys Gln Ala Ser
1               5                   10